(19) Europäisches Patentamt
European Patent Office
Office européen des brevets

(11) EP 0 673 947 B1

(12) EUROPEAN PATENT SPECIFICATION

(45) Date of publication and mention of the grant of the patent: **12.07.2000 Bulletin 2000/28**

(21) Application number: **94902104.2**

(22) Date of filing: **10.12.1993**

(51) Int. Cl.$^7$: **C07H 7/027**, C07H 9/04, C07H 15/04, C07H 15/18

(86) International application number: **PCT/JP93/01796**

(87) International publication number: **WO 94/13685 (23.06.1994 Gazette 1994/14)**

# (54) PSICOFURANOSE AND PSICOPYRANOSE DERIVATIVES

PSICOFURANOSE UND PSICOPYRANOSE DERIVATE

DERIVES PSICOFURANOSE ET PSICOPYRANOSE

(84) Designated Contracting States:
**AT BE CH DE DK ES FR GB GR IE IT LI LU MC NL PT SE**

(30) Priority: **11.12.1992 JP 35230192**

(43) Date of publication of application: **27.09.1995 Bulletin 1995/39**

(73) Proprietor:
**Sankyo Company Limited**
**Tokyo (JP)**

(72) Inventors:
- **TERASHIMA, Shiro**
  **Tokyo 156 (JP)**
- **KATOH, Tadashi**
  **9-1, Minamidai 1-chome**
  **Kanagawa 228 (JP)**
- **MATSUMOTO, Miyoko**
  **Tokyo 194 (JP)**

(74) Representative:
**Gibson, Christian John Robert et al**
**MARKS & CLERK,**
**57/60 Lincoln's Inn Fields**
**London WC2A 3LS (GB)**

(56) References cited:
- **TETRAHEDRON LETTERS, vol. 34, no. 39, 24 September 1993, OXFORD GB, pages 6289-6292, XP002017251 M.MATSUMOTO ET AL.: "A Novel Biogenetic Type Synthesis of (+)-Hydantocidin."**
- **Tetrahedron, Vol. 47, No. 12/13, p. 2133-2144, March 25, 1991 (25.03.91), S. MIO et al., "Refer to Synthetic Studies of (+)- Hydantocidin (3)", p. 2134, Scheme-2, and p. 2139, Compound 4 Comprises R5, R6, R8 Representing Hydroxylic Protecting Groups in Claim 3. Compound 6 Equivalent to One where R7 is Hydrogen and X Protecting Hydroxymethyl Group is Equivalent to One where R1-R4 are Hydroxylic Protecting Groups and X Protected Hydroxymethyl Group.**
- **Collection of Czechoslovak Chemical Communication, Vol. 39, No. 4, p. 1098-1106, (1974).**
- **H. HREBABECKY et al., "Synthesis of 1-Beta-D-Psicofuranoxylvracil and 1-Beta-D-Psicofuranosyl Cytosine", Methyl Groups and Toluic Groups Refer to Compounds of Expressions II, III in p. 1099, are Equivalent to Hydroxylic Protecting Groups.**
- **Carbohydrate Research, No. 71, p. 149-167 (1979), P.C.M. HERVE DU PENHOAT et al., "Synthesis and Stereo Chemistry of Some Derivertives of D-Psicose", Pyranose and Furanose Derivatives of Psicose are Described, including Hydroxylic Protecting Groups such as Isopropylidene Groups and Acetyl Groups.**
- **Journal of Chemical Society, Perkin Transaction 1, No. 12, p. 3353-3357 (1988).**
- **A. BOSCHETTI et al., "Synthesis of Keto-Furanoses by Epoxidation-Ring Closure of Enol Ethers", Benzyl Ether Derivatives of D-Psicofuranose are Described.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

### Technical field

This invention relates to a key synthetic intermediate in a novel process for preparing a substance having a potent herbicidal activity, hydantocidin represented by the following formula.

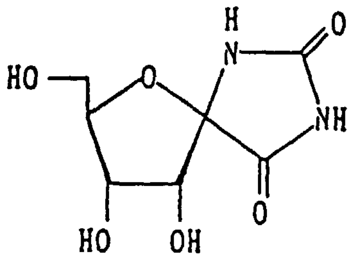

—— [ I ]

### Background art

Hydantocidin represented by the above formula [I], which is obtained as a metabolite of actinomycetes, has excellent transferability to plants and potent grass-killing activity and growth control activity to both annual and perennial monocotyledonous and dicotyledonous weeds. Further, hydantocidin shows remarkable safety to animals, fish and microorganisms and also it has environmentally desirable properties such as high degradability in soil, so that it is a compound which is expected to be used as an excellent herbicide. It is considered that when hydantocidin is industrially prepared by extraction from a metabolite of actinomycetes, column chromatography or the like would inevitably be used repeatedly for separating hydantocidin from amino acid, nucleic acid, etc. which are admixed therewith, thus causing great difficulties [M. Nakajima et al., J. Antibiot., 44, 293 (1991)]. Thus, as a conventional method of synthesizing hydantocidin, there have been reported (1) a synthesis in which a spiro-ring cyclization reaction is performed using a tartaric acid derivative and a hydantoin derivative as starting substances [S. Mio et al., Tetrahedron, 47, 2111, 2121 (1991) and Japanese Provisional Patent Publication (Kokai) No. Hei 2-85287], (2) a synthetic method using a N-glycosylation reaction of a D-psicose derivative obtained from D-fructose [S. Mio et al., Tetrahedron, 47, 2133 (1991)] and (3) a synthetic method in which a hydantoin-forming reaction is performed using a D-ribose derivative as a starting substance [S. Mirza, German Patent Laid-Open Publication DE 4129728 A1 (1991)]. However, a large number of reagents have been used which are expensive and have high toxicity, i.e. osmium tetraoxide and lithium hexamethyldisilazide in the method of (1), trimethylsilylazide in the method of (2) and trifluoromethanesulfonic anhydride and osmium tetraoxide in the method of (3). Thus, industrial preparation of hydantocidin by these synthetic methods has involved great difficulties.

The present inventors made intensive studies on development of a process for industrially preparing hydantocidin which is a substance having an excellent herbicidal activity, and consequently found that a D-psicofuranose derivative and a D-psicopyranose derivative which are the compounds of the present invention are extremely useful key preparation intermediates, to accomplish the present invention.

### Disclosure of the invention

The present invention relates to a D-psicofuranose derivative represented by the following formula:

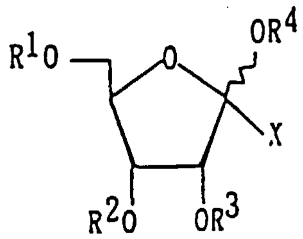

[II]

(wherein $R^1$, $R^2$, $R^3$ and $R^4$ may be the same or different from one another and each represents a hydrogen atom or a protective group for a hydroxyl group, and X represents a hydroxymethyl group, carboxyl group, carbamoyl group or allophanoyl group, which may be protected),and a D-psicopyranose derivative represented by the following formula:

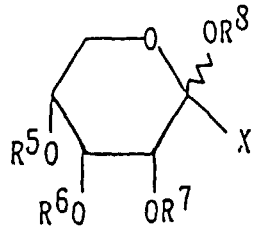

—— [III]

(wherein $R^5$, $R^6$, $R^7$ and $R^8$ may be the same or different from one another and each represents a hydrogen atom or a protective group for a hydroxyl group, and X represents a hydroxymethyl group, carboxyl group, carbamoyl group or allophanoyl group, which may be protected).

The protective group for a hydroxyl group may be exemplified by an arylmethyl group such as a benzyl group, a p-methoxybenzyl group, a 2,4-dimethoxybenzyl group, a p-chlorobenzyl group, a m-bromobenzyl group, a p-nitrobenzyl group, a benzhydryl group, a di-p-anisylmethyl group and a trityl group; a silyl group such as a trimethylsilyl group, a triethylsilyl group, a tripropylsilyl group and a t-butyldimethylsilyl group; an alkyl group such as a methyl group, an ethyl group, an allyl group, a methoxymethyl group, a 2-methoxyethoxymethyl group, a benzyloxymethyl group, a methylthiomethyl group, a 2,2,2-trichloroethoxymethyl group, a 2-(trimethylsilyl)ethoxymethyl group, a tetrahydropyranyl group and a 1-ethoxyethyl group; an acyl group such as a formyl group, an acetyl group, a trifluoroacetyl group, a pivaloyl group, a benzoyl group, a p-methoxybenzoyl group, a p-chlorobenzoyl group, a methoxycarbonyl group, an ethoxycarbonyl group, a benzyloxycarbonyl group and a t-butoxycarbonyl group, etc.

Further, protective groups for two hydroxyl groups of $R^2$ and $R^3$, $R^5$ and $R^6$ or $R^6$ and $R^7$ which are adjacent to each other may together represent an alkylidene or arylmethylidene group represented by the following formula:

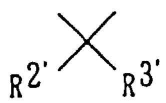

—— [IV]

(wherein $R^{2'}$ and $R^{3'}$ may be the same or different from each other and each represent a hydrogen atom, a straight or branched alkyl group having 1 to 6 carbon atoms or a substituted or unsubstituted aryl group, and may together form a ring). Examples thereof may include an alkylidene group such as an isopropylidene group, a 1-methylpropylidene group, a 1-methylbutylidene group, a cyclopentylidene group and a cyclohexylidene group; an arylmethylidene group such as a benzylidene group, a p-methoxybenzylidene group, a p-chlorobenzylidene group, a 1-methylbenzylidene group, a 1-ethylbenzylidene group, a diphenylmethylidene group, etc.

In the above formula [II], when $R^2$ and $R^3$ are protective groups for hydroxyl groups, it is preferred that $R^2$ and $R^3$ together represent the alkylidene or arylmethylidene group represented by the above formula [IV].

A protected hydroxymethyl group refers to a hydroxymethyl group in which a hydroxyl group thereof is substituted by the protective group for a hydroxyl group such as an arylmethyl group, a silyl group, an alkyl group, an acyl group, etc, which is described above. A protected carboxyl group refers to a carboxyl group which is esterified by a lower alkyl group, a silyl group or the like, or a carboxyl group which is neutralized by an alkali metal salt, an amine or the like. Further, a protected carbamoyl group or allophanoyl group refers to a carbamoyl or allophanoyl group in which a nitrogen atom thereof is substituted by an arylmethyl group, a silyl group, an alkyl group, an acyl group or the like, which is the same as the above protective group for a hydroxyl group.

The straight or branched alkyl group having 1 to 6 carbon atoms may be exemplified by a methyl group, an ethyl group, a propyl group, a butyl group, an isobutyl group, a pentyl group, an isopentyl group, a hexyl group. etc. The substituted or unsubstituted aryl group may be exemplified by a phenyl group, a p-tolyl group, a p-methoxyphenyl group, a m-chlorophenyl group, a naphthyl group, a furyl group, a thienyl group, etc.

The psicofuranose and psicopyranose derivatives represented by the above formulae [II] and [III] can be prepared by the following synthetic steps, respectively.

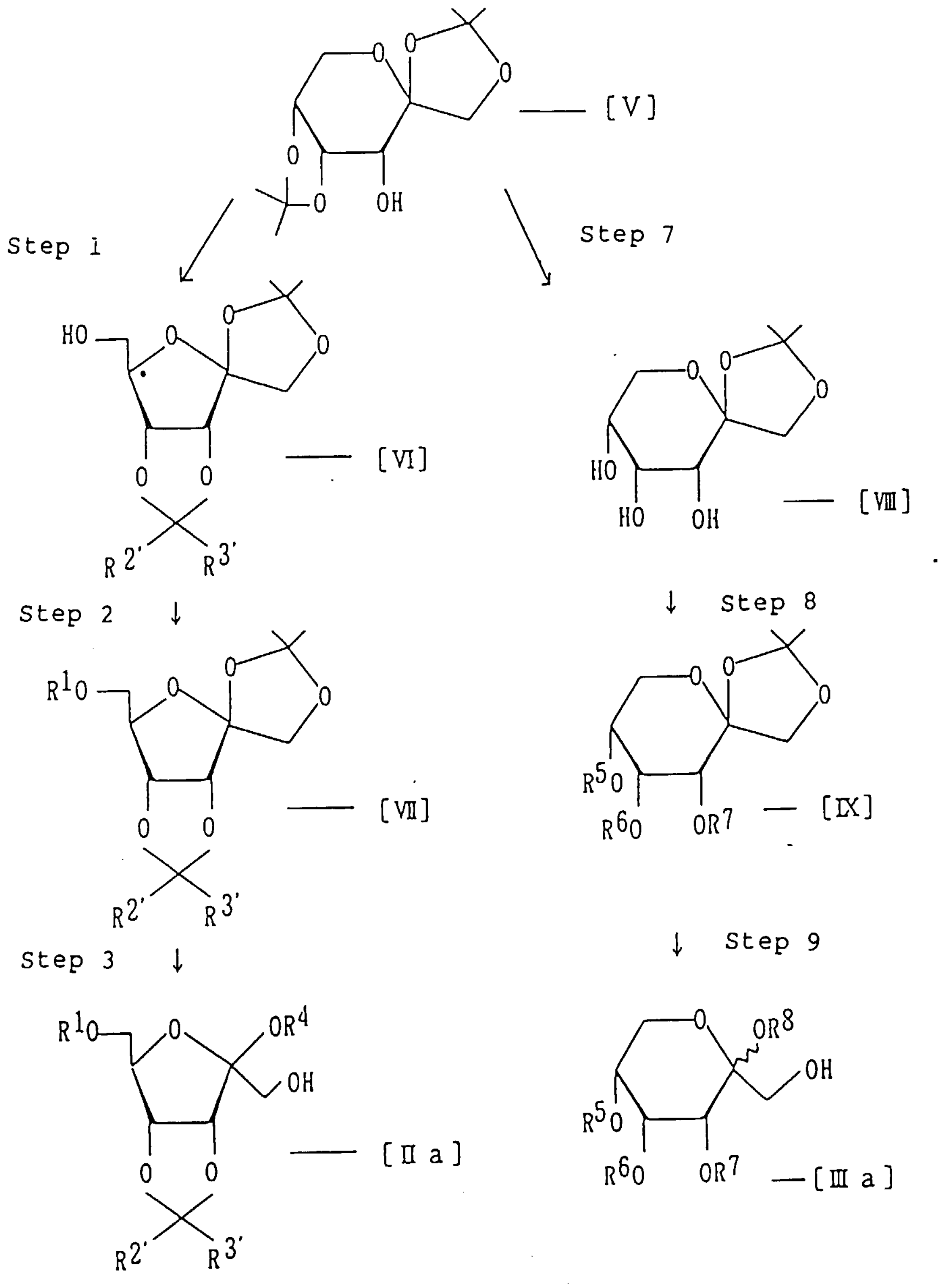


[V]
[VI]
[VII]
[II a]
[VIII]
[IX]
[III a]

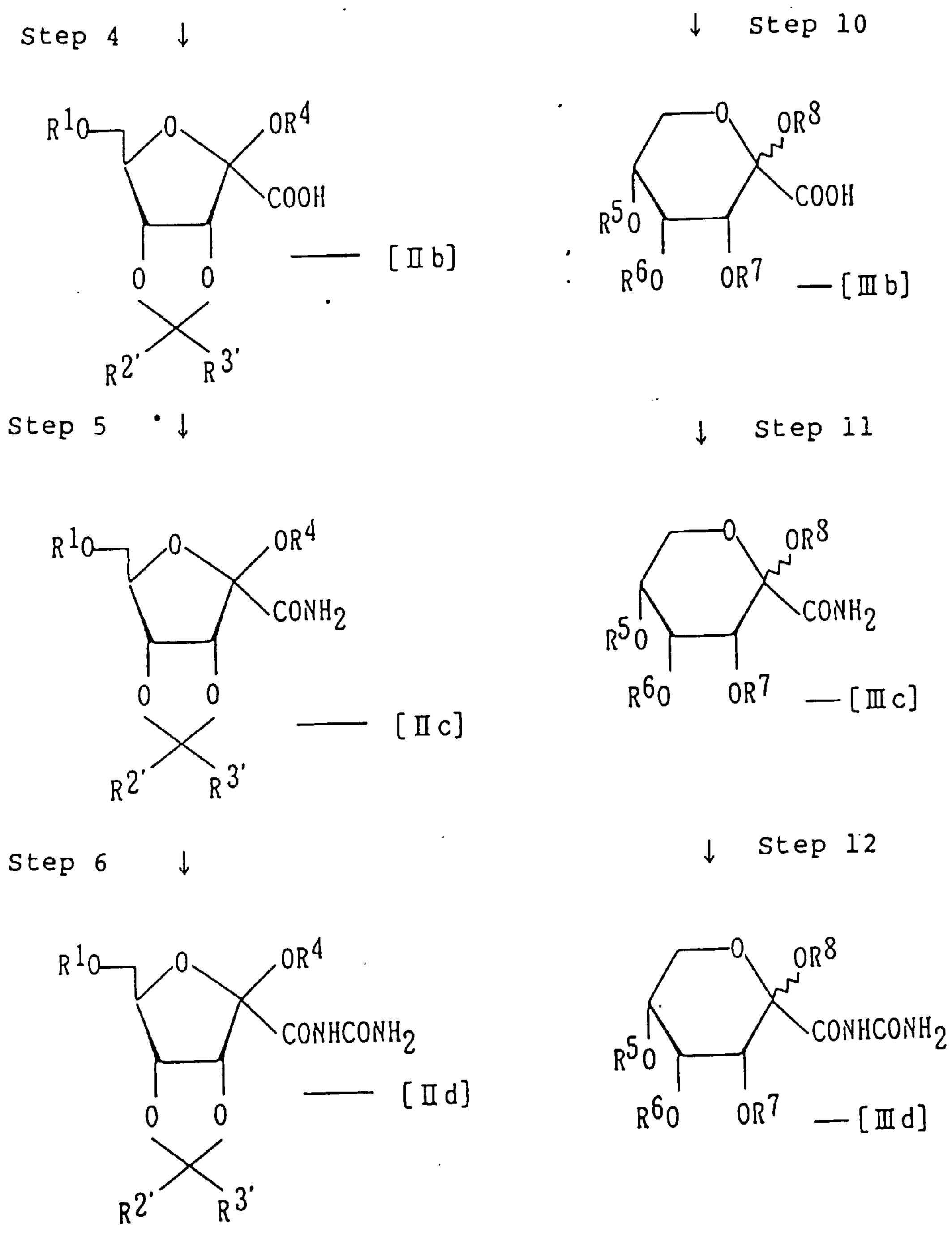


[IIb] [IIIb] [IIc] [IIIc] [IId] [IIId]

(wherein $R^1$, $R^2$, $R^3$, $R^4$, $R^5$, $R^6$, $R^7$, $R^8$, $R^{2'}$ and $R^{3'}$ each have the same meanings as described above).

[Step 1]

In this step. 1,2:4,5-di-O-isopropylidene-β-psicopyranose represented by the formula [V] reacts with an acetal derivative in the presence of an acid catalyst to prepare a D-psicofuranose derivative represented by the formula [VI] in which hydroxyl groups at 1- and 2-positions are protected by isopropylidene groups and hydroxyl groups at 3- and 4-positions are protected by alkylidene groups or arylmethylidene groups. The 1,2:4,5-di-O-isopropylidene-D-psicopyranose represented by the formula [V] to be used in this step is a compound which can be prepared from D-fructose according to a method described in literature [S. Mio et al., Tetrahedron, 47, 2133 (1991)].

The acetal derivative to be used in this step is exemplified by 2,2-dimethoxypropane, 2,2-diethoxypropane,

2,2-dipropoxypropane, 2,2-dimethoxypropane, 2,2-diethoxybutane, 3,3-dimethoxypentane, 3,3-diethoxypentane, 1,1-dimethoxycyclopentane, 1,1-diethoxycyclopentane, 1,1-dimethoxycyclohexane, 1,1-diethoxycyclohexane, benzaldehyde dimethylacetal, benzaldehyde diethylacetal, p-methoxybenzaldehyde dimethylacetal, p-chlorobenzaldehyde dimethylacetal, benzophenone dimethylacetal, acetophenone dimethylacetal, propiophenone dimethylacetal, etc., and 2,2-dimethoxypropane is preferably used. The acid catalyst to be used in this step is exemplified by hydrochloric acid, sulfuric acid, nitric acid, perchloric acid, hydrofluoric acid, hydrobromic acid, acetic acid, trifluoroacetic acid, methanesulfonic acid, p-toluenesulfonic acid, trifluoromethanesulfonic acid, fluorosulfonic acid, pyridinium p-toluenesulfonate, etc., and perchloric acid is preferably used.

The reaction is carried out in a solvent. The solvent to be used may be any solvent so long as it does not participate in the reaction, and there may be preferably used ketone-type solvents such as acetone, 2-butanone, 3-pentanone, cyclopentanone, cyclohexanone, etc., ether-type solvents such as ethyl ether, tetrahydrofuran, dioxane, etc. and halogenated hydrocarbon type solvents such as dichloromethane, chloroform, 1,2-dichloroethane, etc., and a ketone type solvent corresponding to the acetal derivative to be used is more preferably used. The reaction proceeds smoothly at -20°C to 50°C.

[Step 2]

In this step, a protective group is introduced into the hydroxyl group at the 6-position of the D-psicofuranose derivative represented by the formula [VI], in which hydroxyl groups at 1- and 2-positions and 3- and 4-positions are protected, to prepare a D-psicofuranose derivative represented by the formula [VII] in which all hydroxyl groups at 1-, 2-, 3-, 4- and 6-positions are protected.

As the protective group for a hydroxyl group to be introduced in this step, there is selected a protective group which can exist stably from Step 3 to Step 6 subsequent to this step, and which can be removed easily without modifying other moieties of the compound in Step 13 described later. The protective group for a hydroxyl group which satisfies such requirements is exemplified by an arylmethyl group such as a benzyl group, a p-methoxybenzyl group, a 2,4-dimethoxybenzyl group, a p-chlorobenzyl group, a p-bromobenzyl group, a p-nitrobenzyl group, a benzhydryl group, a di-p-anisylmethyl group, a trityl group, etc.; a silyl group such as a trimethylsilyl group, a triethylsilyl group, a tripropylsilyl group, a t-butyldimethylsilyl group, etc.; an alkyl group such as a methyl group, an ethyl group, an allyl group, a methoxymethyl group, a 2-methoxyethoxymethyl group, a benzyloxymethyl group, a methylthiomethyl group, a 2,2,2-trichloroethoxymethyl group, a 2-(trimethylsilyl)ethoxymethyl group, a tetrahydropyranyl group, a 1-ethoxyethyl group, etc.; and an acyl group such as a formyl group, an acetyl group, a trifluoroacetyl group, a pivaloyl group, a benzoyl group, a p-methoxybenzoyl group, a p-chlorobenzoyl group, a methoxycarbonyl group, an ethoxycarbonyl group, a benzyloxycarbonyl group, a t-butoxycarbonyl group, etc. An arylmethyl group is preferably used, and a benzyl group is more preferably used. These protective groups for hydroxyl groups are introduced by a known method [T.W. Green, "Protective groups in Organic Synthesis" A Wiley-Interscience Publication, New York, 1981, pp. 10 to 72].

[Step 3]

In this step, the D-psicofuranose derivative represented by the formula [VII] in which hydroxyl groups at 1- and 2-positions are protected by isopropylidene group reacts with an alcohol in the presence of an acid catalyst to prepare a D-psicofuranose derivative represented by the formula [IIa] having a hydroxymethyl group, which is a compound of the present invention, in which only the isopropylidene group at 1- and 2-positions is selectively removed and an alkyl residue of the alcohol used is introduced into a hydroxyl group at 2-position as a protective group.

The alcohol to be used in this step is exemplified by methanol, ethanol, propanol, isopropanol, butanol, isobutanol, cyclopentanol, cyclohexanol, benzyl alcohol, p-methoxybenzyl alcohol, p-chlorobenzyl alcohol, diphenylmethanol, etc., and benzyl alcohol is preferably used. The acid catalyst to be used in this step is exemplified by hydrochloric acid, sulfuric acid, nitric acid, perchloric acid, hydrofluoric acid, hydrobromic acid, acetic acid, trifluoroacetic acid, methanesulfonic acid, p-toluenesulfonic acid, trifluoromethanesulfonic acid, fluorosulfonic acid, pyridinium p-toluenesulfonate, etc., and hydrochloric acid, p-toluenesulfonic acid, trifluoromethanesulfonic acid, etc. are preferably used.

The reaction is carried out in a solvent. The solvent to be used may be any solvent so long as it does not interfere with the reaction, and there may be preferably used alcohol type solvents such as methanol, ethanol, propanol, isopropanol, butanol, isobutanol, etc., hydrocarbon type solvents such as benzene, toluene, xylene, hexane, pentane, etc., halogenated hydrocarbon type solvents such as dichloromethane, chloroform, 1,2-dichloroethane, etc. and ether type solvents such as ethyl ether, tetrahydrofuran, dioxane, etc., and it is more preferred to use an extremely excessive amount of an alcohol as both the reactant and the solvent. The reaction proceeds smoothly at -20°C to 50°C.

[Step 4]

In this step, the hydroxymethyl group of the D-psicofuranose derivative represented by the formula [IIa] is oxidized to prepare a D-psicofuranose derivative represented by the formula [IIb] having a carboxyl group. Oxidation of the hydroxymethyl group to a carboxyl group can be carried out in one step by oxidation using chromium trioxide-sulfuric acid, oxidation using pyridinium chromate or oxidation using a platinum catalyst, but it is carried out preferably by two-stage oxidation, i.e. oxidizing the hydroxymethyl group to an aldehyde group and then oxidizing the aldehyde group to a carboxyl group. The oxidation of the hydroxymethyl group to the aldehyde group is exemplified by methods generally used for oxidizing a hydroxymethyl group to an aldehyde group, such as oxidation using oxalyl chloride-dimethyl sulfoxide-triethylamine, oxidation using sulfur trioxide pyridine complex-dimethyl sulfoxide-triethylamine. oxidation using chromium trioxide pyridine complex. oxidation using acetic anhydride-dimethyl sulfoxide. oxidation using pyridinium chlorochromate, etc., and oxidation using oxalyl chloride-dimethyl sulfoxide-triethylamine is preferably selected.

The oxidation of the aldehyde group to the carboxyl group which is carried out subsequently is exemplified by methods generally used for oxidizing an aldehyde group to a carboxyl group, such as oxidation using sodium chlorite-sodium dihydrogenphosphate dihydrate, oxidation using chromium trioxide-sulfuric acid, oxidation using pyridinium chromate, air oxidation using a platinum catalyst, etc., and oxidation using sodium chlorite-sodium dihydrogen phosphate dihydrate is preferably selected. The reaction is carried out in a sovlent. The solvent to be used may be any solvent so long as it does not participate in the reaction. When the hydroxymethyl group is oxidized to the carboxyl group in one step, there may be used ketone-type solvents such as acetone and methyl ethyl ketone, halogenated hydrocarbon-type solvents such as dichloromethane, chloroform and 1,2-dichloroethane, etc. When the hydroxymethyl group is oxidized to the carboxyl group through the aldehyde group by two stages, ketone-type solvents such as acetone and methyl ethyl ketone, halogenated hydrocarbon-type solvents such as dichloromethane, chloroform and 1,2-dichloroethane, etc. may be used in the oxidation at the first stage and, in addition to the above solvents, ether-type solvents such as ethyl ether, tetrahydrofuran and dioxane, t-butanol and water as a single solvent or a mixed solvent may be used in the oxidation at the second stage. All of the oxidation reactions proceed smoothly at -20°C to 50°C.

[Step 5]

In this step, the carboxyl group of the D-psicofuranose derivative represented by the formula [IIb] having a carboxyl group is converted into a carbamoyl group to prepare a D-psicofuranose derivative represented by the formula [IIc] having a carbamoyl group, which is a compound of the present invention.

This step is carried out by converting the carboxyl group into a carboxylic acid chloride using thionyl chloride, phosphorus trichloride, phosphorus pentachloride or the like; a mixed acid anhydride of carboxylic acid and carbonic acid half ester using methoxycarbonyl chloride, ethoxycarbonyl chloride, isopropoxycarbonyl chloride or the like and a tertiary amine such as triethylamine, pyridine, etc.; or a carboxylic acid active amide using carbonyldiimidazole or the like; followed by a reaction with ammonia. This step is carried out preferably through a mixed acid anhydride of carboxylic acid and carbonic acid half isopropyl ester obtained using isopropoxycarbonyl chloride and triethylamine. The activation of carboxylic acid and the subsequent amidation are carried out in a solvent. The solvent to be used may be any solvent so long as it does not participate in the reaction, and ether type solvents such as ethyl ether, tetrahydrofuran, dioxane, etc. are preferably used. The activation of carboxylic acid and the subsequent amidation proceed smoothly at -50°C to 50°C.

[Step 6]

In this step, a D-psicofuranose represented by the formula [IId] having an allophanoyl group is prepared from the D-psicofuranose derivative represented by the formula [IIc] having a carbamoyl group .

This step is carried out by converting the furanose derivative having a carbamoyl group into an acylisocyanate derivative using an oxalyl halide such as oxalyl chloride, oxalyl bromide or the like, followed by reaction thereof with ammonia. The acylisocyanate derivative is prepared preferably by using oxalyl chloride. The preparation of the acylisocyanate derivative and the subsequent preparation of the D-psicofuranose derivative having an allophanoyl group are carried out in a solvent. The solvent to be used may be any solvent so long as it does not participate in the reaction, and halogenated hydrocarbon type solvents such as dichloromethane, chloroform, 1,2-dichloroethane, etc. and ether type solvents such as ethyl ether, tetrahydrofuran, dioxane, etc. are preferably used. The preparation of the acylisocyanate derivative proceeds smoothly at -20°C to 120°C, and the preparation of the D-psicofuranose derivative therefrom having an allophanoyl group proceeds smoothly at -20°C to 50°C.

[Step 7]

In this step, the isopropylidene group at the 4- and 5- positions of the 1,2:4,5-di-O-isopropylidene-β-psicopyranose represented by the formula [V] is selectively removed to prepare a 1,2-O-isopropylidene-β-D-psicopyranose represented by the formula [VIII].

The 1,2:4,5-di-O-isopropylidene-β-D-psicopyranose represented by the formula [V] to be used in this step is a compound which can be prepared by a method described in literature [S. Mio et al., Tetrahedron, 47, 2133 (1991)].

The isopropylidene group at the 4- and 5-positions is selectively removed using an acid catalyst. The acid catalyst to be used is exemplified by hydrochloric acid, sulfuric acid, nitric acid, perchloric acid, hydrofluoric acid, hydrobromic acid, acetic acid, trifluoroacetic acid, methanesulfonic acid, p-toluenesulfonic acid, trifluoromethanesulfonic acid, fluorosulfonic acid, pyridinium p-toluenesulfonate, etc., and p-toluenesulfonic acid is preferably used. The reaction is carried out in a solvent. As the solvent to be used, there may be preferably used alcoholic solvents such as methanol, ethanol, propanol, isopropanol, etc. The reaction proceeds smoothly at -20°C to 50°C.

[Step 8]

In this step, the same or different protective groups are introduced to each of the hydroxyl groups at 3-, 4- and 5-positions of the 1,2-O-isopropylidene-β-D-psicopyranose represented by the formula [VIII] to prepare a D-psicopyranose derivative represented by the formula [IX] in which hydroxyl groups at 1-, 2-, 3-, 4- and 5-positions are protected.

As the same or different protective groups for hydroxyl groups to be introduced in this step, there may be selected those which can exist stably from Step 9 to Step 12, and which can be removed without modifying other moieties of the compound in the subsequent Step 14. The protective groups for hydroxyl groups which satisfy such requirements are exemplified by an arylmethyl group such as a benzyl group, a p-methoxybenzyl group, a 2,4-dimethoxybenzyl group, a p-chlorobenzyl group, a p-bromobenzyl group, a p-nitrobenzyl group, a benzhydryl group, a di-p-anisylmethyl group, a trityl group, etc.; a silyl group such as a trimethylsilyl group, a triethylsilyl group, a tripropylsilyl group and a t-butyldimethylsilyl group; an alkyl group such as a methyl group, an ethyl group, an allyl group, a methoxymethyl group, a 2-methoxyethoxymethyl group, a benzyloxymethyl group, a methylthiomethyl group, a 2,2,2-trichloroethoxymethyl group, a 2-(trimethylsilyl)ethoxymethyl group, a tetrahydropyranyl group and a 1-ethoxyethyl group; an acyl group such as a formyl group, an acetyl group, a trifluoroacetyl group, a pivaloyl group, a benzoyl group, a p-methoxybenzoyl group, a p-chlorobenzoyl group, a methoxycarbonyl group, an ethoxycarbonyl group, a benzyloxycarbonyl group and a t-butoxycarbonyl group; an alkylidene group and an arylmethylidene group such as an isopropylidene group, a diethylmethylidene group, a cyclopentylidene group, a cyclohexylidene group, a benzylidene group and a diphenylmethylidene group, etc. An arylmethyl group is preferably used, and a benzyl group is more preferably used. These protective groups are introduced at one time or stepwisely according to a known method [T.W. Green, "Protective groups in Organic Synthesis" A Wiley-Interscience Publication, New York, 1981, pp. 10 to 86].

[Step 9]

In this step, the D-psicopyranose represented by the formula [IX] in which hydroxyl groups at 1- and 2-positions are protected by isopropylidene groups reacts an alcohol in the presence of an acid catalyst to prepare a D-psicopyranose derivative represented by the formula [IIIa] having a hydroxymethyl group, which is a compound of the present invention, in which only the isopropylidene group at 1- and 2-positions is selectively removed and an alkyl residue of the alcohol used is introduced into a hydroxyl group at 2-position as a protective group.

This step is carried out entirely in the same manner as in Step 3, preferably by using benzyl alcohol as an alcohol and hydrochloric acid as an acid catalyst.

[Step 10]

In this step, the primary alcohol existing at 1-position of the D-psicopyranose derivative represented by the formula [IIIa] is oxidized to prepare a D-psicopyranose derivative represented by the formula [IIIb] having a carboxyl group .

This step is carried out entirely in the same manner as in Step 4. In the two-stage oxidation which is preferably carried out, oxidation using oxalyl chloride-dimethyl sulfoxide-triethylamine is preferably used in the oxidation of the hydroxymethyl group to the aldehyde group, and oxidation using sodium chlorite-sodium dihydrogenphosphate dihydrate is preferably used in the oxidation of the aldehyde group to the carboxyl group.

[Step 11]

In this step, the carboxyl group of the D-psicopyranose derivative having a carboxyl group represented by the formula [IIIb] is converted into a carbamoyl group to prepare a D-psicopyranose derivative having a carbamoyl group represented by the formula [IIIc] which is the compound of the present invention.

This step is carried out entirely in the same manner as in Step 5. Carboxylic acid is preferably activated through a mixed acid anhydride of carboxylic acid and carbonic acid half isopropyl ester.

[Step 12]

In this step, a D-psicopyranose derivative represented by the formula [IIId] having an allophanoyl group, which is a compound of the present invention, is prepared from the D-psicopyranose derivative represented by the formula [IIIc] having a carbamoyl group.

This step is carried out entirely in the same manner as in Step 6. The acylisocyanate derivative is preferably prepared using oxalyl chloride.

In all the compounds of the present invention represented by the formulae [IIa] to [IId] and the formulae [IIIa] to [IIId] which can be obtained by the respective steps described above, the protective groups for hydroxyl groups can be removed or the protective groups for hydroxyl groups can be replaced with other protective groups for hydroxyl groups according to a known method.

According to the following synthetic steps, Hydantocidin represented by the formula [I], which is a substance having a herbicidal activity, and 5-epi-hydantocidin represented by the formula [XI], are derived from the D-psicofuranose derivative and D-psicopyranose derivative, each having an allophanoyl group, represented by the formulae [IId] and [IIId] and prepared as described above, according to the following synthetic steps:

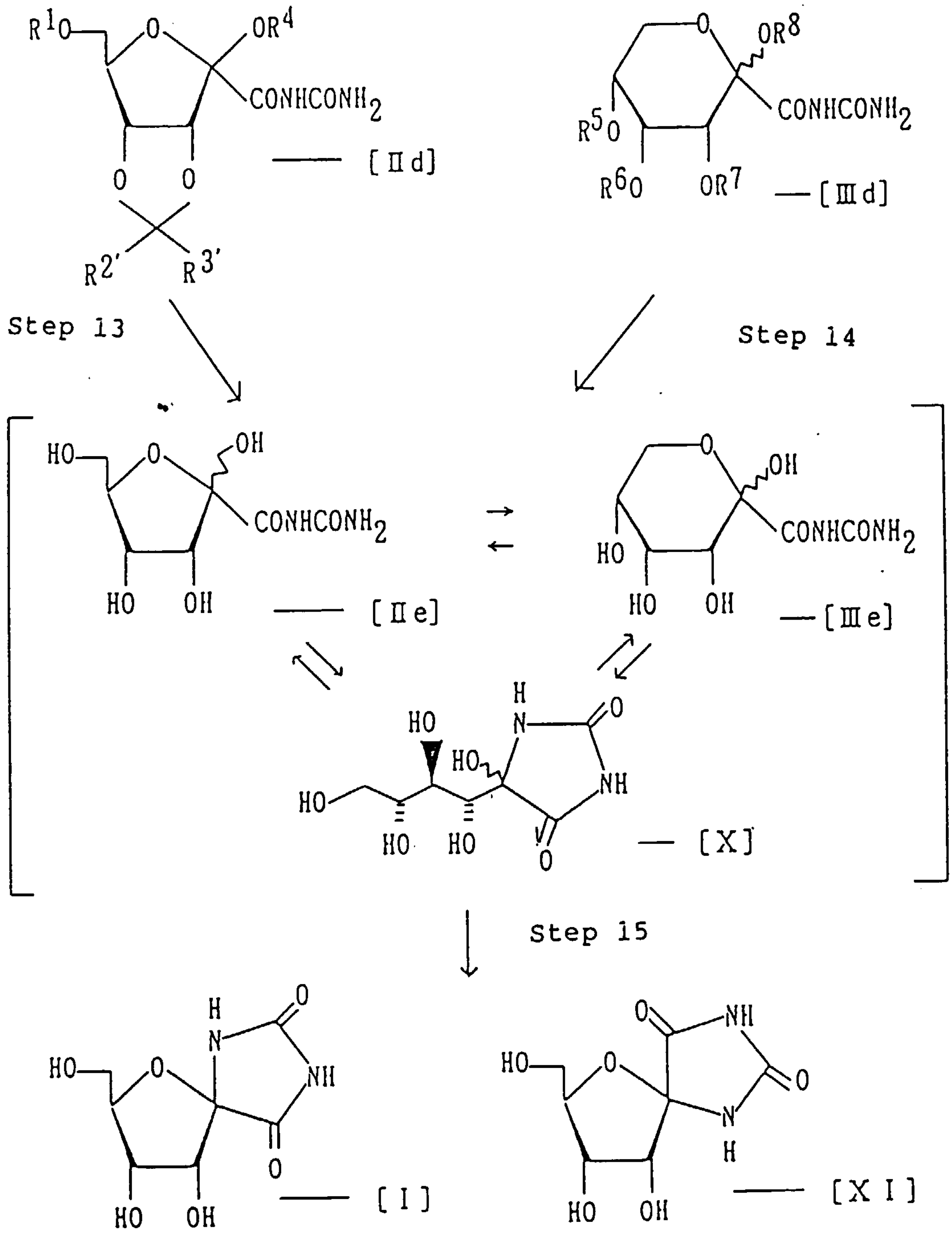


(wherein $R^1$, $R^{2'}$, $R^{3'}$, $R^4$, $R^5$, $R^6$, $R^7$ and $R^8$ have the same meanings as described above).

Thus, by removing the protective groups, a mixture of a D-psicofuranose derivative represented by the formula [IIe] having no protective group, a D-psicopyranose derivative represented by the formula [IIIe] having no protective group and a hydantoin derivative represented by the formula [X] is derived initially. Then, by treating this mixture with an acid catalyst, a mixture of hydantocidin represented by the formula [I] which is a substance having a herbicidal activity and 5-epi-hydantocidin represented by the formula [XI] is derived easily. By leaving to stand for a long time, the above mixture is finally converged to the hydantoin derivative represented by the formula [X]. This [X] also gives a mixture of hydantocidin represented by the formula [I] which is a substance having a herbicidal activity and 5-epi-hydantocidin represented by the formula [XI] by a treatment with an acid catalyst.

[Step 13]

In this step, the hydroxyl protective groups of the D-psicofuranose derivative represented by the formula [IId] having an allophanoyl group are removed to prepare a mixture of the D-psicofuranose derivative, and D-psicopyranose derivative each having an allophanoyl group, represented by the formulae [IIe] and [IIIe], and the hydantoin derivative having no protective group represented by the formula [X].

The protective groups are all removed at one time or stepwisely according to known methods suitable for the protective groups used [T.W. Green, "Protective Groups in Organic Synthesis" A Wiley-Interscience Publication, New York. 1981. pp. 10 to 86].

When the protective group for hydroxyl groups at the 2- and 3-positions ($R^{2'}R^{3'}$ CH<) of the ribofuranose moiety of the D-psicofuranose derivative represented by the formula [IId] having an allophanoyl group is isopropylidene group and the protective groups for hydroxyl groups at 1- and 5-positions ($R^1$ and $R^4$) of the said moiety are benzyl groups, the isopropylidene group is removed by using hydrochloric acid, sulfuric acid, p-toluenesulfonic acid or the like as an acid catalyst in an alcoholic solvent such as methanol, ethanol, propanol, isopropanol, etc., and a D-psicofuranose derivative represented by the formula:

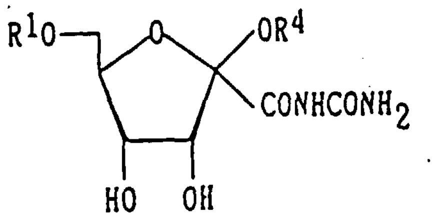

--- [II f]

can be isolated as an intermediate. The benzyl groups are removed from this compound by hydrogenolysis using palladium carried on carbon (palladium-carbon) or the like as a catalyst in an alcoholic solvent such as methanol, ethanol, propanol, isopropanol, etc. As a matter of course, the benzyl groups can be removed without isolating the psicofuranose derivative represented by the formula [IIf].

[Step 14]

In this step, the protective groups for hydroxyl groups of the D-psicopyranose derivative represented by the formula [IIId] having an allophanoyl group are removed to prepare a mixture of the D-psicofuranose derivative and D-psicopyranose derivative, each having no protective group, represented by the formulae [IIe] and [IIIe], and the hydantoin derivative having no protective group represented by the formula [X].

The protective groups are all removed at one time or stepwisely according to known methods suitable for the protective groups used [T.W. Green, "Protective Groups in Organic Synthesis" A Wiley-Interscience Publication, New York, 1981, pp. 10 to 86].

When all the protective groups for hydroxyl groups at 1-, 2-, 3- and 4-positions of the ribopyranose portion of the D-psicopyranose derivative represented by the formula [IIId] having an allophanoyl group are benzyl groups, the benzyl groups are removed by hydrogenolysis using palladium carried on carbon (palladium-carbon) or the like as a catalyst in an alcoholic solvent such as methanol, ethanol, propanol, isopropanol, etc.

[Step 15]

In this step, the mixture of the D-psicofuranose derivative and D-psicopyranose derivative, each having no protective group, represented by the formulae [IIe] and [IIIe], and the hydantoin derivative having no protective group represented by the formula [X], is treated with an acid catalyst to prepare a mixture of hydantocidin represented by the formula [I], which is a substance having a herbicidal activity, and 5-epi-hydantocidin represented by the formula [XI].

The acid catalyst to be used in this step is exemplified by hydrochloric acid, sulfuric acid, nitric acid, perchloric acid, hydrofluoric acid, hydrobromic acid, acetic acid, trifluoroacetic acid, methanesulfonic acid, p-toluenesulfonic acid, trifluoromethanesulfonic acid, fluorosulfonic acid, pyridinium p-toluenesulfonate, etc. and a strongly acidic ion exchange resin such as Dowex 50W, Amberlite IR-120, etc. Dowex 50W which is a strongly acidic ion exchange resin is preferably used. The reaction is carried out in a solvent. The solvent to be used may be any solvent so long as it does not participate in the reaction, and there may be preferably used water, alcoholic solvents such as methanol, ethanol, propanol, isopropanol, etc. or mixed solvents of these alcoholic solvents and water. The reaction proceeds smoothly at

0°C to 50°C.

Hydantocidin represented by the formula [I], which is a substance having a herbicidal activity, and 5-epi-hydantocidin represented by the formula [XI], obtained in this step, can be separated by treating the mixture of both compounds obtained in the reaction with an excess amount of acetic anhydride in pyridine to convert them into the corresponding tetraacetates, and separating a mixture of the tetraacetates by preparative thin layer chromatography using silica gel or the like. Further, by treating the respective separated tetraacetates in methanol with ammonia or hydrazine, hydantocidin represented by the formula [I] and 5-epi-hydantocidin represented by the formula [XI] can be obtained as pure products.

The present invention will be described below specifically by way of Examples and Reference Examples, but it should be noted that the present invention is not limited thereto.

[Reference Example 1]

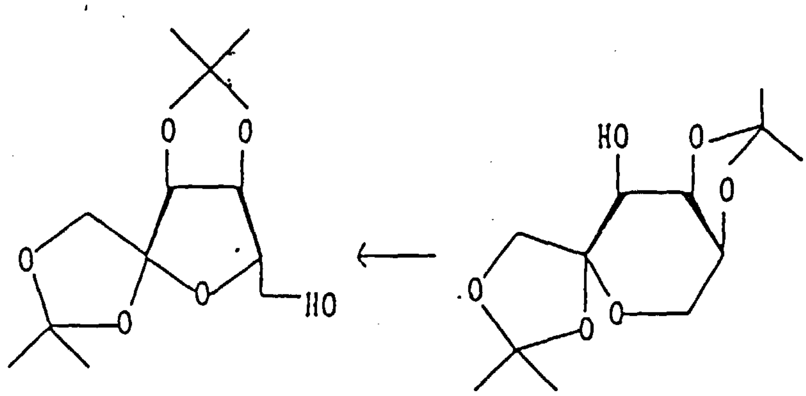

2,2-Dimethoxypropane (33.6 ml, 0.27 mol) was added to a solution of 1,2:4,5-di-O-isopropylidene-α-D-psicopyranose (141 g, 0.54 mol) in acetone (1.4L). After the temperature of the mixture was made 0°C, perchloric acid (a 70% aqueous solution) (5 ml) was added thereto. The mixture was stirred for 4 hours and then the reaction mixture was neutralized with concentrated aqueous ammonia to terminate the reaction. The solvent was concentrated under reduced pressure, the resulting residue was partitioned with water and ether, and then the aqueous layer was extracted with ethyl ether. The organic layers were combined, washed with a saturated aqueous NaCl solution, dried over anhydrous sodium sulfate and filtered. Then, the solvent was evaporated under reduced pressure. The residue was purified by silica gel column chromatography (hexane/ethyl acetate = 85/15) to obtain 1,2:3,4-di-O-isopropylidene-β-D-psicofuranose as a white solid (99.0 g, 0.38 mol, 70%).

$^1$H NMR(200MHZ, $CDCl_3$) δ

1.33 (3H, s, Me)
1.41 (3H, s, Me)
1.45 (3H,s, Me)
1.51 (3H,s, Me)
3.64 (1H, dd, J=3.5, 12.5Hz, H-6)
3.77 (1H, dd, J=2.4, 12.5Hz, H-6)
4.07 (1H, d, J=9.8Hz, H-1)
4.28-4.34 (1H, m, H-5)
4.34 (1H, d, J=9.8Hz, H-1)
4.65 (1H, d, J=5.9Hz, H-3)
4.92 (1H, dd, J=1.0, 5.9Hz, H-4)

[Reference Example 2]

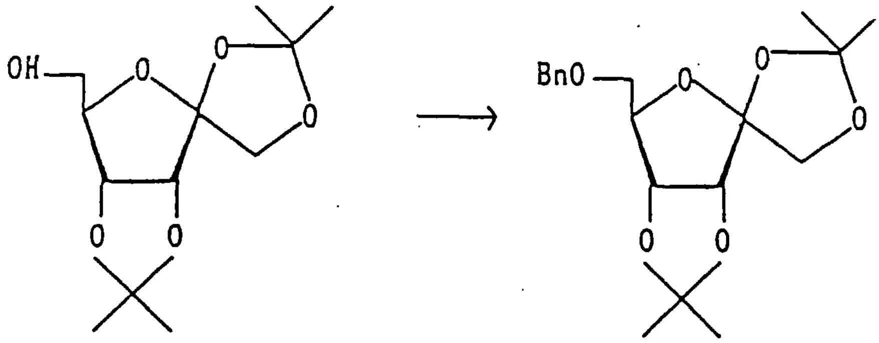

An aqueous solution (272 ml) of benzyltriethylammonium chloride (4.76 g, 0.021 mol) and sodium hydroxide (139 g, 3.5 mol) was added to a mixture of 1,2:3,4-di-O-isopropylidene-β-D-psicofuranose (90.7 g, 0.35 mol) and benzyl chloride (160 ml), followed by vigorous stirring at 100°C for 2 hours. The reaction mixture was partitioned with water and ether, and the aqueous layer was extracted with ethyl ether. The organic layers were combined, washed with a saturated aqueous NaCl solution, dried over anhydrous sodium sulfate and filtered. Then, the solvent was evaporated under reduced pressure. The residue was purified by silica gel column chromatography (hexane/ethyl ether = 95/5 and then hexane/ethyl acetate = 9/1) to obtain 6-O-benzyl-1,2:3,4-di-O-isopropylidene-β-D-psicofuranose as a pale yellow oil (113 g, 92%).

$^1$H NMR(200MHz, $CDCl_3$) δ

1.32 (3H, s, Me)
1.37 (3H, s, Me)
1.43 (3H, s, Me)
1.44 (3H, s, Me)
3.52 (1H, dd, J=8.2, 9.8Hz, H-6)
3.59 (1H, dd, J=6.3, 9.8Hz, H-6)
4.04 (1H, d, J=9.6Hz, H-1)
4.29 (1H, d, J=9.6Hz, H-1)
4.27-4.35 (1H, m, H-5)
4.52-4.65 (3H, m, -C<u>H</u>$_2$Ph, H-3)
4.76 (1H, dd, J=1.0, 5.9, H-4)
7.27-7.42 (5H, m)

[Example 1]

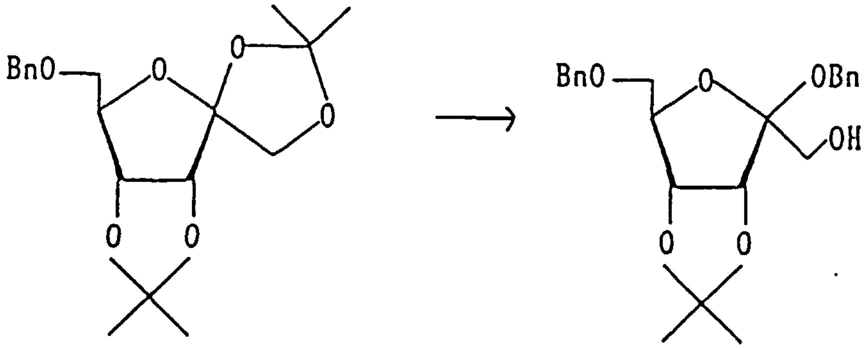

Trifluoromethanesulfonic acid (2 ml) was slowly added dropwise at room temperature to a solution of 6-O-benzyl-1,2:3,4-di-O-isopropylidene-β-D-psicofuranose (39.5 g, 0.11 mol) in benzyl alcohol (350 ml). The mixture was stirred for 2 hours and then neutralized with concentrated aqueous ammonia to terminate the reaction. Benzyl alcohol was evaporated under reduced pressure. The residue was partitioned with ethyl acetate and water, and the aqueous layer was extracted with ethyl acetate. The organic layers were combined, washed with a saturated aqueous NaCl solution, dried over anhydrous sodium sulfate and filtered. Then, the solvent was evaporated under reduced pressure. The residue was purified by silica gel column chromatography (hexane/ethyl acetate = 8/2) to obtain benzyl 6-O-benzyl-3,4-O-isopropylidene-β-D-psicofuranoside as a pale yellow oil (27.9 g, 62%).

$[\alpha]_D^{20}$ -29.8° (c 1.55, $CHCl_3$)
IR (neat, $cm^{-1}$)

3520, 2950, 1450, 1390, 1370, 1220, 1180, 1140, 1110

$^1$H NMR(400MHz, $CDCl_3$) δ

1.33 (3H, s, Me)
1.53 (3H, s, Me)
1.95 (1H, br, OH)
3.47 (1H, dd, J=9.6, 7.3Hz, H-6)
3.52 (1H, dd, J=9.6, 7.1Hz, H-6)
3.82-3.94 (2H, m, H-1)
4.42 (1H, ddd, J=7.3, 7.1, 1.4Hz, H-5)
4.44 (1H, d, J=12.1Hz, -C$\underline{H}_2$Ph)
4.49 (1H, d, J=12.1Hz, -C$\underline{H}_2$Ph)
4.54 (1H, d, J=11.8Hz, -C$\underline{H}_2$Ph)
4.61 (1H, d, J=11.8Hz, -C$\underline{H}_2$Ph)
4.68 (1H, d, J=6.1Hz, H-3)
4.72 (1H, dd, J=6.1, 1.4Hz, H-4)
7.25-7.36 (10H, m)

HRMS (m/z)

| | | |
|---|---|---|
| $M^+$-$CH_2OH(C_{22}H_{25}O_5)$ | Found | 369.1679 |
| | Calcd. | 369.1690 |

[Example 2]

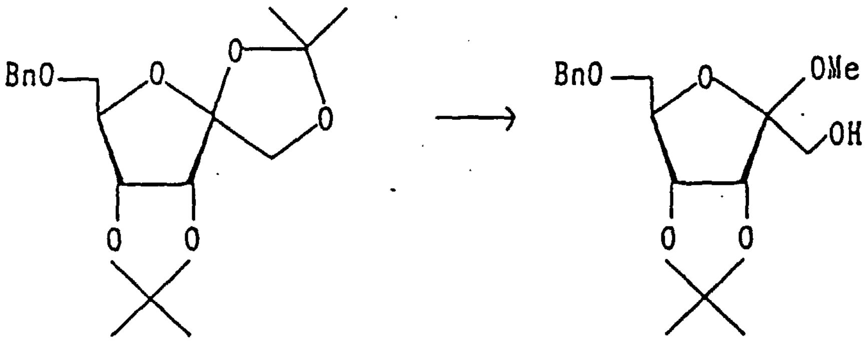

Methanol in which hydrogen chloride had been saturated at 25°C (a solution of 43% hydrogen chloride in methanol: 0.25 ml) was added at 0°C to a solution of 6-O-benzyl-1,2:3,4-di-O-isopropylidene-β-D-psicopyranose (25.3 mg, 0.072 mmol) dissolved in methanol (0.75 ml). The mixture was stirred at room temperature for 25 minutes and then poured into a mixed solution of ice-saturated aqueous solution of sodium hydrogencarbonate-ethyl acetate to terminate the reaction. The reaction mixture was partitioned with ethyl acetate and water, and the aqueous layer was extracted with ethyl acetate. The organic layers were combined, washed with a saturated aqueous NaCl solution, dried over anhydrous sodium sulfate, filtered and then concentrated under reduced pressure. The residue was purified by silica gel column chromatography (hexane/ethyl acetate = 55/45) to obtain methyl 6-O-benzyl-3,4-O-isopropylidene-β-D-psicofuranoside as a colorless oil (12.5 mg, 53%).

$^1$H NMR(200MHz, $CDCl_3$) δ

1.33 (3H, s, Me)
1.51 (3H, s, Me)
1.97 (1H, brt, J=6.6Hz, OH)
3.23 (3H, s, OMe)
3.48 (1H, dd, J=7.4, 9.6Hz, H-6)
3.55 (1H, dd, J=7.0, 9.6Hz, H-6)
3.77 (1H, brdd, J=6.9, 12.2Hz, H-1)
3.85 (1H, brdd, J=5.9, 12.2Hz, H-1)
4.38 (1H, ddd, J=1.4, 7.0, 7.4Hz, H-5)
4.56 (1H, d, J=6.0Hz, H-3)
4.57 (2H, s, -C$\underline{H}_2$Ph)
4.71 (1H, dd, J=1.4, 6.0Hz, H-4)
7.28-7.38 (5H, m, Ar-H)

[Example 3]

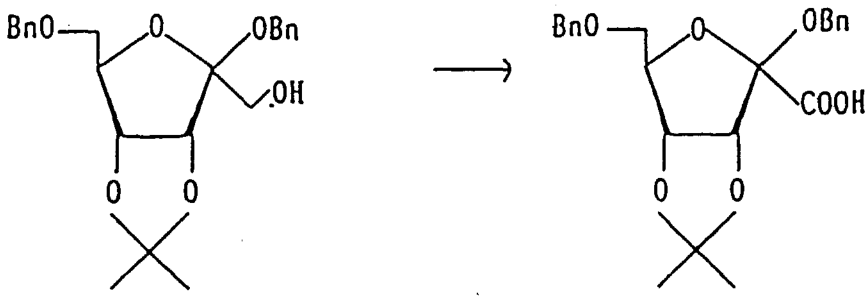

Dimethyl sulfoxide (24.7 ml, 0.35 mol) was added dropwise at -78°C to a solution of oxalyl chloride (15.2 ml, 0.17 mol) in methylene chloride (150 ml). After the mixture was stirred for 10 minutes, a solution of benzyl 6-O-benzyl-3,4-O-isopropylidene-β-D-psicopyranoside (27.9 g, 0.070 mol) dissolved in methylene chloride (50 ml) was added thereto, followed by further stirring for 20 minutes. Next, triethylamine (67.9 ml, 0.49 mol) was added to the mixture, the temperature of the resulting mixture was raised to 0°C over 45 minutes, and then a saturated aqueous sodium hydrogencarbonate solution was poured into the mixture to terminate the reaction. The reaction mixture was partitioned with ethyl acetate and water, and the aqueous layer was extracted with ethyl acetate. The organic layers were combined, washed with a saturated aqueous NaCl solution, dried over anhydrous sodium sulfate and concentrated under reduced pressure to obtain 2,6-di-O-benzyl-1-didehydro-3,4-isopropylidene-β-D-psicofuranose as a crude product.

Next, 2,6-di-O-benzyl-1-didehydro-3,4-isopropylidene-β-D-psicofuranose was dissolved in a mixed solvent of a t-butanol (300 ml) and water (90 ml). To the resulting solution were added 2-methyl-2-butene (36.9 ml, 0.35 mol) and sodium dihydrogenphosphate dihydrate (43.6 g, 0.28 mol). While sufficiently stirring the mixture, sodium chlorite (25.3 g, 0.28 ml) was gradually added thereto. The mixture was stirred at room temperature for 1 hour and then concentrated under reduced pressure until the amount of t-butanol became about a third of the solvent amount, followed by partition with ethyl ether and water. The aqueous layer was extracted with ethyl ether, and the organic layers were combined, washed successively with 2% hydrochloric acid and a saturated aqueous NaCl solution, dried over anhydrous sodium sulfate and concentrated under reduced pressure to obtain crude 1,5-di-O-benzyl-1-α-carboxy-1-dehydro-2,3-O-isopropylidene-β-D-ribofuranose as a white solid (30.0 g, quantitative yield).

$^1$H NMR(400MHz, $CD_3OD$) δ

1.33 (3H, s, Me)
1.50 (3H, s, Me)
3.47-3.55 (1H, brd, H-5)
3.55-3.62 (1H, brd, H-5)
4.40-4.92 (7H, m, H-2, H-3, H-4, -C$\underline{H}_2$Ph)
7.25-7.38 (10H, Ar-H)

This carboxylic acid had low solubility in various solvents and analysis thereof by proton NMR spectrum was difficult, so that its structure was confirmed by further converting it to methyl ester. That is, one drop of conc. hydrochloric acid was added to a solution of 1,5-di-O-benzyl-1-α-carboxy-1-dehydro-2,3-O-isopropylidene-β-D-ribofuranose (18.0 mg, 0.015 mmol) in methanol (2 ml), followed by stirring at 50°C for 24 hours. The mixture was neutralized by adding conc. aqueous ammonia to terminate the reaction and concentrated under reduced pressure. The residue was purified by preparative silica gel thin layer chromatography (hexane/ethyl acetate = 3/7) to obtain 1,5-di-O-benzyl-1-α-methoxycarbonyl-1-dehydro-β-D-ribofuranose (11.4 mg, 68%).

$[\alpha]_D^{20}$ -46.7° (c 1.02, $CHCl_3$)
$^1$H NMR(400MHz, $CDCl_3$) δ

2.65 (1H, brd, J=8.7Hz, OH-3)

3.40 (1H, brd, J=3.5Hz, OH-2)
3.66 (1H, dd, J=4.8, J=10.3Hz, H-5)
3.70 (1H, dd, J=4.7, J=10.3Hz, H-5)
3.80 (3H, s, COOMe)
4.30 (1H, brdd, J=3.5, 4.6Hz, H-2)
4.34 (1H, ddd, J=4.7, 4.8, 7.0Hz, H-4)
4.38 (1H, d, J=11.2Hz, -C<u>H</u>$_2$Ph)
4.68 (1H, brddd, J=4.6, 7.0, 8.7Hz, H-3)
4.56 (2H, s, -C<u>H</u>$_2$Ph)
4.69 (1H, d, J=11.2Hz, -C<u>H</u>$_2$Ph)
7.33-7.20 (10H, m, Ar-H)

[Example 4]

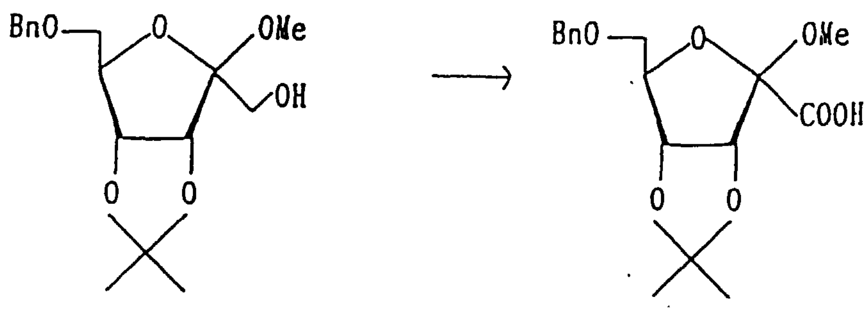

Dimethyl sulfoxide (72.9 µl, 1.0 mmol) was added dropwise at -78°C to a solution of oxalyl chloride (44.9 µl, 0.52 mmol) dissolved in methylene chloride (2 ml). After the mixture was stirred for 15 minutes, a solution of methyl 6-O-benzyl-3,4-O-isopropylidene-β-D-psicofuranoside (41.7 mg, 0.13 mmol) in methylene chloride (2 ml) was added thereto, and the resulting mixture was further stirred for 15 minutes. Next, triethylamine (0.179 ml, 1.3 mol) was added to the mixture, the temperature of the resulting mixture was raised to 0°C over 2 hours, and then a saturated aqueous sodium hydrogencarbonate solution was poured into the mixture to terminate the reaction. The reaction mixture was partitioned with ethyl acetate and water, and the aqueous layer was extracted with ethyl acetate. The organic layers were combined, washed with a saturated aqueous NaCl solution, dried over anhydrous sodium sulfate and concentrated under reduced pressure to obtain 6-O-benzyl-1-didehydro-3,4-isopropylidene-2-O-methyl-β-D-psicofuranose as a crude product.

Next, 2-methyl-2-butene (68.0 µl, 0.64 mmol) was added to a solution of 6-O-benzyl-1-didehydro-3,4-isopropylidene-2-O-methyl-β-D-psicofuranose in t-butanol (1 ml). To the mixture were further added sodium dihydrogenphosphate dihydrate (17.5 mg, 0.19 mmol) and an aqueous sodium chlorite (30.1 mg, 0.19 mmol) (0.4 ml). The mixture was stirred at room temperature for 30 minutes and then partitioned with ether and water, and the aqueous layer was extracted with ether. The organic layers were combined, washed successively with 2% hydrochloric acid and a saturated aqueous NaCl solution, dried over anhydrous sodium sulfate and concentrated under reduced pressure to obtain crude 5-O-benzyl-1-α-carboxy-1-dehydro-2,3-O-isopropylidene-1-O-methyl-β-D-ribofuranose as a colorless oil (44.7 mg, quantitative yield).

$^1$H NMR(200MHz, CDCl$_3$) δ

1.28 (3H, brs, Me)
1.46 (3H, brs, Me)
3.18 (3H, brs, OMe)
3.46-3.68 (2H, br, H-5)
4.50-4.84 (5H, br, H-2,3,4, -C<u>H</u>$_2$Ph)
7.28-7.42 (5H, m, Ar-H)

[Example 5]

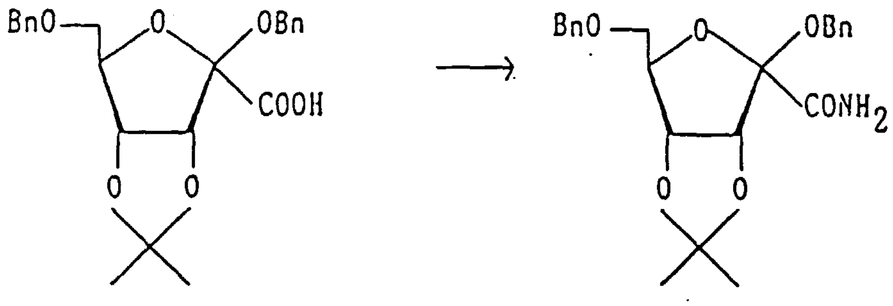

Triethylamine (38.8 ml, 0.28 mol) was added to a solution of 1,5-di-O-benzyl-1-α-carboxy-1-dehydro-2,3-O-isopropylidene-β-D-ribofuranose (30.0 g, 0.070 mol) in tetrahydrofuran (350 ml). The mixture was cooled to 0°C, and isopropoxycarbonyl chloride (24.4 ml, 0.21 mol) was slowly added dropwise thereto. The mixture was stirred at 0°C for 30 minutes and at room temperature for 30 minutes. After disappearance of the starting compounds was confirmed, ammonia gas was blown into the mixture at 0°C, and the mixture was further stirred at room temperature for 30 minutes. The mixture was concentrated under reduced pressure until the amount of tetrahydrofuran became about a third of the solvent amount. The concentrate was partitioned with methylene chloride and water, and the aqueous layer was extracted with methylene chloride. The organic layers were combined, washed with a saturated aqueous NaCl solution and dried over anhydrous sodium sulfate, filtered and then concentrated under reduced pressure. The residue was purified by silica gel column chromatography (hexane/ethyl acetate = 45/55) to obtain 1,5-di-O-benzyl-1-α-carbamoyl-1-dehydro-2,3-O-isopropylidene-β-D-ribofuranose as white needle crystals (26.5 g, yield from benzyl 6-O-benzyl-3,4-O-isopropylidene-β-D-psicopyranoside: 92 %).

m.p. 145-146°
$[\alpha]_D^{20}$ -37.4° (c 1.23, $CHCl_3$)
IR (KBr, $cm^{-1}$)

3480, 3280, 2970, 1720, 1690, 1670, 1460, 1380, 1250, 1220, 1100

$^1$H NMR(400MHz, $CDCl_3$) δ

1.29 (3H, s, Me)
1.49 (3H, s, Me)
3.48 (1H, dd, J=9.7, 7.0Hz, H-5)
3.55 (1H, dd, J=9.7, 7.7Hz, H-5)
4.43 (1H, d, J=12.0Hz, -C<u>H</u>$_2$Ph)
4.48 (1H, d, J=10.7Hz, -C<u>H</u>$_2$Ph)
4.50 (1H, d, J=12.0Hz, -C<u>H</u>$_2$Ph)
4.54 (1H, d, J=10.7Hz, -C<u>H</u>$_2$Ph)
4.56 (1H, ddd, J=7.7, 7.0, 1.2Hz, H-4)
4.68 (1H, dd, J=5.8, 1.2Hz, H-3)
4.83 (1H, d, J=5.8Hz, H-2)
5.83 (1H, brs, N<u>H</u>)
6.73 (1H, brs, N<u>H</u>) 7.26-7.36 (10H, m, Ar-H)

HRMS (m/z)

| | | |
|---|---|---|
| $M^+$-Me($C_{22}H_{24}N_1O_6$) | Found | 398.1602 |
| | Calcd. | 398.1602 |

[Example 6]

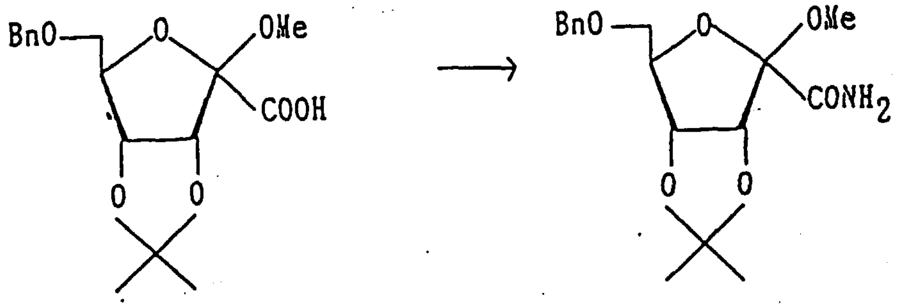

Triethylamine (35.9 μl, 0.26 mmol) was added to a solution of 5-O-benzyl-1-α-carboxy-1-dehydro-2,3-O-isopropylidene-1-O-methyl-β-D-ribofuranose (44.7 mg, 0.13 mmol) in tetrahydrofuran (2 ml). The mixture was cooled to 0°C, and isopropoxycarbonyl chloride (18.3 μl, 0.42 mol) was slowly added dropwise thereto. The mixture was stirred at 0°C for 30 minutes and at room temperature for 30 minutes. After disappearance of the starting compounds was confirmed, ammonia gas was blown into the mixture at 0°C, and the mixture was further stirred at room temperature for 30 minutes. The mixture was partitioned with methylene chloride and water, and the aqueous layer was extracted with methylene chloride. The organic layers were combined, washed successively with diluted hydrochloric acid and a saturated aqueous NaCl solution, dried over anhydrous sodium sulfate, filtered and then concentrated under reduced pressure. The residue was purified by preparative silica gel thin layer chromatography (hexane/ethyl acetate = 3/7) to obtain 5-O-benzyl-1-α-carbamoyl-1-dehydro-2,3-O-isopropylidene-1-O-methyl-β-D-ribofuranose as a colorless oil (33.7 mg, yield from methyl 6-O-benzyl-3,4-O-isopropylidene-β-D-psicopyranoside: 78%).

$^1$H NMR(200MHz, $CDCl_3$) δ

1.29 (3H, s, Me)
1.47 (3H, s, Me)
3.23 (3H, s, OMe)
3.51 (1H, dd, J=9.7, 7.0Hz, H-5)
3.58 (1H, dd, J=9.7, 7.7Hz, H-5)
4.53 (1H, ddd, J=7.7, 7.0, 0.9Hz, H-4)
4.57 (2H, s, -C<u>H</u>$_2$Ph)
4.68 (1H, dd, J=5.9, 0.9Hz, H-3)
4.73 (1H, d, J=5.9Hz, H-2)
5.67 (1H, brs, NH)
6.65 (1H, brs, NH)
7.29-7.38 (5H, m, Ar-H)

[Example 7]

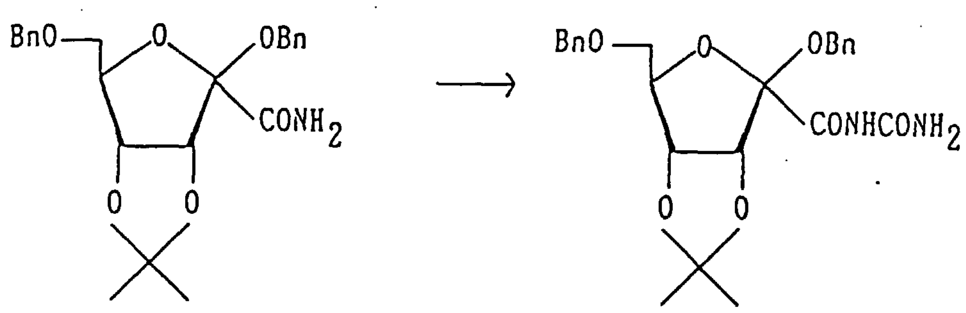

Oxalyl chloride (7.31 ml, 84 mmol) was added at room temperature to a solution of 1,5-di-O-benzyl-1-α-carbamoyl-1-dehydro-2,3-O-isopropylidene-β-D-ribofuranose (17.3 g, 42 mmol) in 1,2-dichloroethane (500 ml), and the mixture was stirred for 30 minutes. After the mixture was further stirred at 90°C for 2 hours, the reaction mixture was cooled to room temperature and stirred for 30 minutes while blowing ammonia gas thereinto. The reaction mixture was partitioned with methylene chloride and water, and the aqueous layer was extracted with methylene chloride. The organic layers were combined, washed with a saturated aqueous NaCl solution, dried over anhydrous sodium sulfate, filtered and then concentrated under reduced pressure. The residue was purified by silica gel column chromatography (hexane/ethyl acetate = 1/1) to obtain 1,5-di-O-benzyl-1-α-allophanoyl-1-dehydro-2,3-O-isopropylidene-β-D-ribofuranose as a colorless oil (16.6 g, 87%).

$[\alpha]_D^{20}$ -68.1° (c 1.06, $CHCl_3$)
IR (neat, $cm^{-1}$)

3450, 3050, 1730, 1580, 1480, 1460, 1380, 1220, 1100

$^1H$ NMR(400MHz, $CDCl_3$) δ

1.28 (3H, s, Me)
1.47 (3H, s, Me)
3.48 (1H, dd, J=9.8, 7.2Hz, H-5)
3.56 (1H, dd, J=9.8, 7.3Hz, H-5)
4.38 (1H, d, J=10.8Hz, -C$\underline{H}_2$Ph)
4.46 (1H, d, J=12.0Hz, -C$\underline{H}_2$Ph)
4.51 (1H, d, J=12.0Hz, -C$\underline{H}_2$Ph)
4.54 (1H, d, J=10.8Hz, -C$\underline{H}_2$Ph)
4.63 (1H, ddd, J=7.3, 7.2, 1.0Hz, H-4)
4.72 (1H, dd, J=5.8, 1.0Hz, H-3)
4.81 (1H, d, J=5.8Hz, H-2)
5.39 (1H, brs, NH)
7.26-7,36 (10H, m, Ar-H)
8.05 (1H, brs, NH)
8.64 (1H, brs, NH)

HRMS (m/z)

| | | |
|---|---|---|
| $M^+-CH_3(C_{23}H_{25}N_2O_7)$ | Found | 441.1661 |
| | Calcd. | 441.1660 |

CIMS (m/z)

457 ($M^+$+1)

[Example 8]

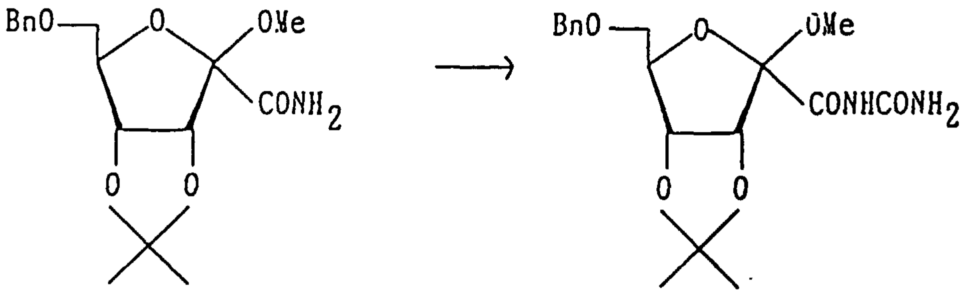

Oxalyl chloride (17.5 µl, 0.20 mmol) was added at room temperature to a solution of 5-O-benzyl-1-α-carbamoyl-1-dehydro-2,3-O-isopropylidene-1-O-methyl-β-D-ribofuranose (33.7 mg, 0.10 mmol) in 1,2-dichloroethane (2 ml), and the mixture was stirred for 30 minutes. After the mixture was stirred at 90°C for 3.5 hours, the reaction mixture was cooled to room temperature. Further, oxalyl chloride (8.7 µl, 0.10 mmol) was added to the mixture, followed by stirring at 90°C for 1.5 hours. The reaction mixture was cooled to room temperature again and then stirred for 30 minutes while blowing ammonia gas thereinto. The reaction mixture was partitioned with methylene chloride and water, and the aqueous layer was extracted with methylene chloride. The organic layers were combined, washed with a saturated aqueous NaCl solution, dried over anhydrous sodium sulfate, filtered and then concentrated under reduced pressure. The residue was purified by preparative silica gel thin layer chromatography (hexane/ethyl acetate = 4/6) to obtain 5-O-benzyl-1-α-allophanoyl-1-dehydro-2,3-O-isopropylidene-1-O-methyl-β-D-ribofuranose as a colorless oil (31.4 mg, 83%).

$^1H$ NMR(200MHz, $CDCl_3$) δ

1.28 (3H, s, Me)
1.46 (3H, s, Me)
3.20 (3H, s, OMe)
3.51 (1H, dd, J=9.7, 7.1Hz, H-5)
3.58 (1H, dd, J=9.7, 7.1Hz, H-5)
4.52-4.67 (3H, m)
4.72 (2H, AB)
5.59 (1H, brs, NH)
7.29-7.40 (5H, m, Ar-H)
8.04 (1H, brs, NH)
8.61 (1H, brs, NH)

$^1H$ NMR(200MHz, $CDCl_3$) δ

3.17 (3H, s, OMe)

3.47-3.73 (3H, m, H-5, OH)
4.12-4.45 (4H, m, H-2,3,4, OH)
4.57 (2H, AB, -C<u>H</u>$_2$Ph)
5.95 (1H, brs, NH)
7.27-7.38 (5H, m, Ar-H)
8.04 (1H, brs, NH)
9.00 (1H, brs, NH)

[Reference Example 3]

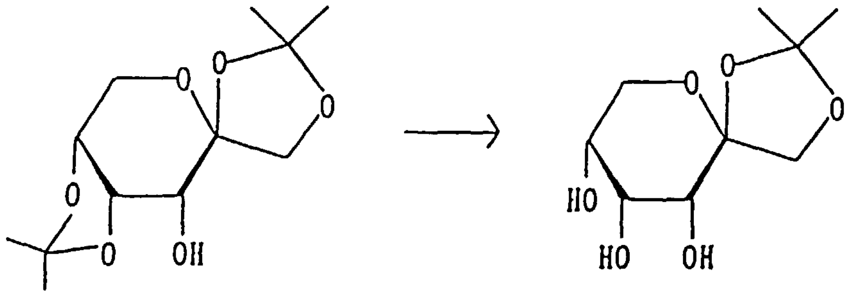

P-toluenesulfonic acid (294 mg, 1.6 mmol) was added at room temperature to a solution of 1,2:4,5-di-O-isopropylidene-β-D-psicopyranose (8.03 g, 31 mmol) in methanol (20 ml), and the mixture was stirred for 1 hour. As the reaction proceeded, a product was precipitated. After disappearance of the starting compounds was confirmed, the mixture was neutralized with concentrated aqueous ammonia to terminate the reaction. The precipitates were filtered to obtain 1,2-O-isopropylidene-β-D-psicopyranose as white mica-like crystals (5.83 g, 86%).

m.p. 174-176°
$[\alpha]_D^{20}$ -111° (C 0.83, $H_2O$)
IR (KBr, $cm^{-1}$)

3430, 3330, 2950, 1075

$^1H$ NMR(400MHz, $D_2O$) δ

1.42 (3H, s, Me)
1.52 (3H, s, Me)
3.78-3.85 (2H, m)
3.92-3.99 (3H, m)
4.13 (1H, d, J=9.9Hz, H-6)
4.17 (1H, d, J=9.9Hz, H-6)

| Elemental analysis | | | |
|---|---|---|---|
| $C_9H_{16}O_6$ | Found | C, 48.82; | H, 7.18 |
| | Calcd. | C, 49.09; | H, 7.32 |

[Reference Example 4]

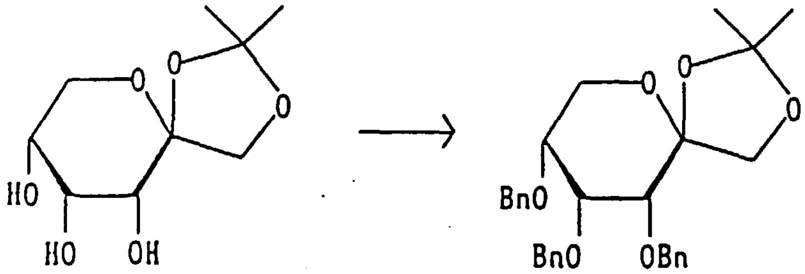

1,2-O-isopropylidene-β-D-psicopyranose (1.90 g, 8.6 mmol) was suspended in benzyl chloride (16 ml), and finely pulverized potassium hydroxide (9.25 g, 17 mmol) was added thereto, followed by vigorous stirring at 130°C for 3 hours. The reaction mixture was cooled to room temperature and then partitioned with chloroform and water, and the aqueous layer was extracted with chloroform. The organic layers were combined, washed successively with water and a saturated aqueous NaCl solution, dried over anhydrous sodium sulfate, filtered and then concentrated under reduced pressure. The residue was purified by silica gel column chromatography (hexane/ethyl acetate = 95/5 and then 7/3) to obtain 3,4,5-tri-O-benzyl-1,2-O-isopropylidene-β-D-psicopyranose as a colorless oil (4.23 g, 100%).

$[\alpha]_D^{20}$ +1° (c 7.78, $CHCl_3$)
IR (neat, $cm^{-1}$)

3000, 2900, 1530, 1475, 1395, 1225, 1070

$^1H$ NMR(400MHz, $CDCl_3$) δ

1.38 (3H, s, Me)
1.51 (3H, s, Me)
3.52 (1H, dt, J=2.4, 7.1Hz, H-5)
3.55 (1H, d, J=2.4Hz, H-3)
3.81 (2H, d, J=7.1Hz, H-6)
4.03 (1H, t, J=2.4Hz, H-4)
4.17 (1H, d, J=9.9Hz, H-1)
4.21 (1H, d, J=9.9Hz, H-1)
4.52 (2H, s, -C$\underline{H}_2$Ph)
4.68 (1H, d, J=11.9Hz, -C$\underline{H}_2$Ph)
4.75 (1H, d, J=12.0Hz, -C$\underline{H}_2$Ph)
4.79 (1H, d, J=12.0Hz, -C$\underline{H}_2$Ph)
4.82 (1H, d, J=11.9Hz, -C$\underline{H}_2$Ph)
7.26-7.39 (15 , m, Ar-H)

HRMS (m/z)

| | | |
|---|---|---|
| $M^+$ ($C_{30}H_{34}O_6$) | Found | 490.2331 |
| | Calcd. | 490.2352 |

[Example 9]

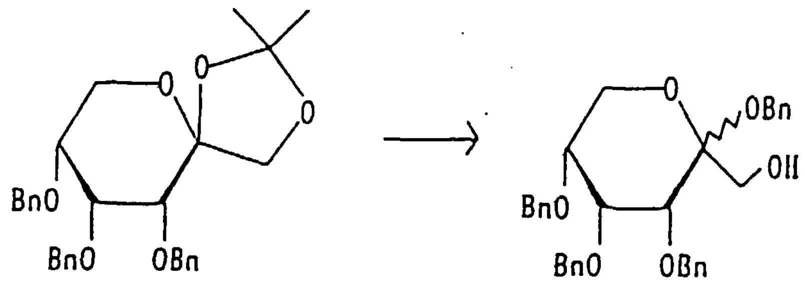

Benzyl alcohol in which hydrogen chloride had been saturated at 0°C (2 ml) was added at 0°C to a solution of 3,4,5-tri-O-benzyl-1,2-O-isopropylidene-β-D-psicopyranose (210 mg, 0.43 mmol) in benzyl alcohol (2 ml). After the mixture was stirred at room temperature for 15 minutes, ammonia gas was blown thereinto at 0°C to terminate the reaction. The reaction mixture was partitioned with ethyl acetate and water, and the aqueous layer was extracted with ethyl acetate. The organic layers were combined, washed with a saturated aqueous NaCl solution, dried over anhydrous sodium sulfate, filtered and then concentrated under reduced pressure. Benzyl alcohol was then distilled off under reduced pressure using a Kugelrohr distillation device. The residue was purified by preparative silica gel thin layer chromatography (hexane/ethyl acetate = 65/35) to obtain benzyl 3,4,5-tri-O-benzyl-D-psicopyranoside as a colorless oil (71.7 mg, 31%).

$[\alpha]_D^{20}$ -80.2° (c 1.34, $CHCl_3$)
IR (neat, $cm^{-1}$)

3500, 3050, 2900, 1450, 1130, 1060

$^1$H NMR(400MHz, $CDCl_3$) δ

1.59 (1H, dd, J=8.3, 5.0Hz, -OH)
3.63 (1H, dd, J=12.3, 2.3Hz, H-6)
3.71-3.77 (2H, m, H-1, H-5)
3.93 (1H, dd, J=3.0, 1.0Hz, H-3)
3.97 (1H, dd, J=12.3, 2.7Hz, H-6)
4.00 (1H, t, J=3.1Hz, H-4)
4.06 (1H, dd, J=12.2, 8.3Hz, H-1)
4.53 (1H, d, J=12.0Hz, $-C\underline{H}_2Ph$)
4.58 (1H, d, J=12.0Hz, $-C\underline{H}_2Ph$)
4.59 (1H, d, J=12.0Hz, $-C\underline{H}_2Ph$)
4.61 (1H, d, J=12.0Hz, $-C\underline{H}_2Ph$)
4.73 (1H, d, J=12.6Hz, $-C\underline{H}_2Ph$)
4.83 (1H, d, J=11.7Hz, $-C\underline{H}_2Ph$)
4.85 (1H, d, J=12.6Hz, $-C\underline{H}_2Ph$)
5.00 (1H, d, J=11.7Hz, $-C\underline{H}_2Ph$)
7.23-7.43 (20H, m)

HRMS (m/z)

| | | |
|---|---|---|
| $M^+-CH_2OH$ ($C_{33}H_{33}O_5$) | Found | 509.2326 |
| | Calcd. | 509.2326 |

[Example 10]

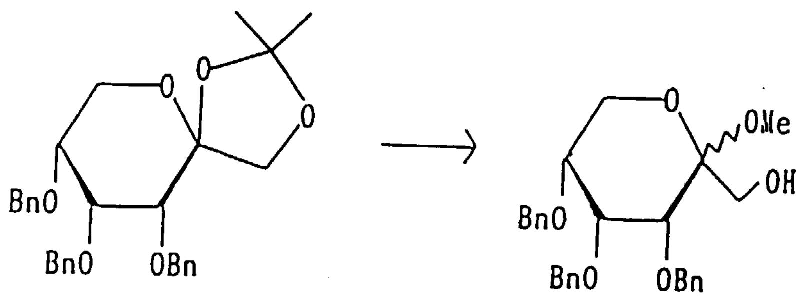

Methanol in which hydrogen chloride had been saturated at 25°C (a solution of 43% hydrogen chloride in methanol: 5 ml) was added at 0°C to a solution of 3,4,5-tri-O-benzyl-1,2-O-isopropylidene-β-D-psicopyranose (1.77 g, 3.6 mmol) in methanol (5 ml). The mixture was stirred at room temperature for 30 minutes and then poured into a mixed solution of ice-saturated aqueous sodium hydrogencarbonate solution-ethyl acetate to terminate the reaction. The reaction mixture was partitioned with ethyl acetate and water, and the aqueous layer was extracted with ethyl acetate. The organic layers were combined, washed with a saturated aqueous NaCl solution, dried over anhydrous sodium sulfate, filtered and then concentrated under reduced pressure. The residue was purified by silica gel column chromatography (hexane/ethyl acetate = 55/45) to obtain methyl 3,4,5-tri-O-benzyl-α,β-D-psicopyranoside as a colorless oil (1.56 g, 93%).

$^1$H NMR(90MHz, $CDCl_3$) δ

7.15-7.64 (15H, m, Ar-H)
4.47-4.99 (6H, m, -C$\underline{H}_2$Ph)
3.39-4.13 (7H, m, H-1,3,4,5,6)
3.23 (3H, s, OMe)

[Example 11]

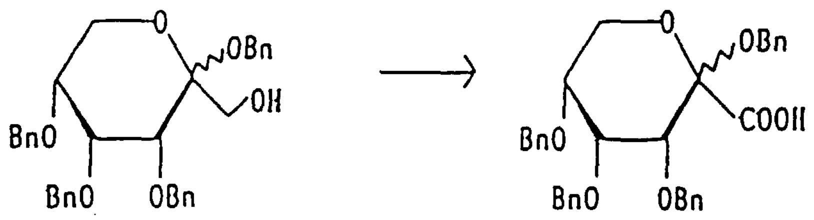

Dimethyl sulfoxide (0.280 ml, 4.0 mmol) was added dropwise at -78°C to a solution of oxalyl chloride (0.173 ml, 2.0 mmol) in methylene chloride (2 ml). After the mixture was stirred for 10 minutes, a solution of benzyl 3,4,5-tri-O-benzyl-D-psicopyranoside (267 mg, 0.49 mmol) in methylene chloride (3 ml) was added thereto, and the resulting mixture was stirred for 25 minutes. Triethylamine (0.687 ml, 4.9 mmol) was then added to the mixture, and the temperature of the resulting mixture was raised to room temperature over 1 hour. A saturated aqueous sodium hydrogencarbonate solution was poured into the mixture to terminate the reaction. The reaction mixture was partitioned with ethyl acetate and water, and the aqueous layer was extracted with ethyl acetate. The organic layers were combined, washed with a

saturated aqueous NaCl solution, dried over anhydrous sodium sulfate and concentrated under reduced pressure to obtain 2,3,4,5-tetra-O-benzyl-1-didehydro-D-psicopyranose as a crude product.

Next, 2-methyl-2-butene (0.522 ml, 4.9 mmol) was added to a solution of 2,3,4,5-tetra-O-benzyl-1-didehydro-D-psicopyranose in t-butanol (2.5 ml). To the mixture were added dropwise an aqueous solution (1 ml) of sodium chlorite (134 mg, 1.5 mmol) and sodium dihydrogenphosphate dihydrate (231 mg, 1.5 mmol). After the mixture was stirred at room temperature for 1 hour, the reaction mixture was partitioned with ethyl ether and water, and the aqueous layer was extracted with ethyl ether. The organic layers were combined, washed successively with an aqueous 2% hydrochloric acid solution and a saturated aqueous NaCl solution, dried over anhydrous sodium sulfate, filtered and concentrated under reduced pressure to obtain crude 1,2,3,4-tetra-O-benzyl-1-carboxy-1-dehydro-D-ribopyranose as a colorless solid (295 mg, quantitative yield).

$^1$H NMR(400MHz, $CDCl_3$) $\delta$

3.38-3.50 (1H, br)
3.58-3.64 (1H, br)
3.67-3.75 (1H, br)
3.78-4.02 (1H, br)
4.17 (1H, brd)
4.30 (2H, brs, -C<u>H</u>$_2$Ph)
4.59-4.90 (6H, m, -C<u>H</u>$_2$Ph)

[Example 12]

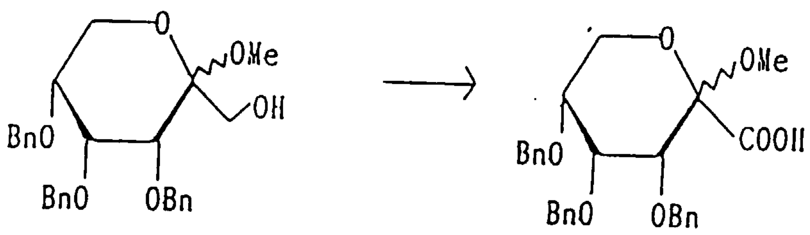

Trimethylamine (0.8 ml) and sulfur trioxide-pyridine complex (95.9 mg, 0.60 mmol) were successively added at 0°C to a solution of methyl 3,4,5-tri-O-benzyl-α,β-D-psicopyranoside (255 mg, 0.55 mmol) in dimethyl sulfoxide (2 ml), and the mixture was stirred at room temperature. After 40 minutes and after 1.5 hours, the same amount as described above of sulfur trioxide-pyridine complex was added, respectively, and the mixture was stirred until the starting compounds disappeared (4 hours). The reaction solution was diluted with ethyl ether, and water was poured thereinto under ice cooling to terminate the reaction. The reaction mixture was partitioned with ethyl ether and water, and the aqueous layer was extracted with ethyl ether. The organic layers were combined, washed successively with an aqueous ammonium choride, a saturated aqueous sodium hydrogencarbonate and a saturated aqueous NaCl solution, dried over anhydrous sodium sulfate and concentrated under reduced pressure to obtain crude 3,4,5-tri-O-benzyl-1-didehydro-2-O-methyl-α,β-D-pysicopyranose. 2-Methyl-2-butene (0.290 ml, 2.7 mmol) was added to the resulting solution of 3,4,5-tri-O-benzyl-1-didehydro-2-O-methyl-α,β-D-pysicopyranose dissolved in t-butanol (4 ml). To the mixture were added dropwise an aqueous solution (1.6 ml) of sodium chlorite (74.4 mg, 0.82 mmol) and sodium dihydrogenphosphate dihydrate (128 mg, 0.82 mmol). After the mixture was stirred at room temperature for 20 minutes, the reaction mixture was partitioned with ethyl ether and water, and the aqueous layer was extracted with ethyl ether. The organic layers were combined, washed successively with a 2% aqueous hydrochloric acid and a saturated aqueous NaCl solution, dried over anhydrous sodium sulfate, filtered and concentrated under reduced pressure. The resulting residue was purified by preparative silica gel thin layer chromatography (chloroform/methanol = 9/1) to obtain 2,3,4-tri-O-benzyl-1-carboxy-1-dehydro-1-O-methyl-α,β-D-ribopyranose as a white solid (222 mg, 85% by two stages).

IR (neat, $cm^{-1}$)

1630, 1100, 1050

$^1H$ NMR(200MHz, $CDCl_3$) δ

7.05-7.40 (15H, m, Ar-H)
4.60-4.90 (4H, br, -C$\underline{H}_2$Ph)
4.25-4.35 (2H, br, -C$\underline{H}_2$Ph)
4.15-4.20 (1H, br)
3.80-4.05 (1H, br)
3.35-3.75 (3H, br)
3.05 (3H, brs, OMe)

CI-MS (m/z)

497($M^+$+H)
447($M^+$-OMe)
433($M^+$-COOH)

This carboxylic acid had low solubility in various solvents and analysis thereof by proton NMR spectroscopy was difficult, so that its structure was confirmed by further converting to its methyl ester. That is, one drop of conc. hydrochloric acid was added to a solution of 2,3,4-tri-O-benzyl-1-carboxy-1-dehydro-1-O-methyl-α,β-D-ribopyranose (23.1 mg, 0.048 mmol) in methanol (2 ml). The mixture was stirred at 90°C for 24 hours. The mixture was neutralized by adding concentrated aqueous ammonia to terminate the reaction and concentrated under reduced pressure. The residue was purified by preparative silica gel thin layer chromatography (hexane/ethyl acetate = 6/4) to obtain 2,3,4-tri-O-benzyl-1-methoxycarbonyl-1-dehydro-1-O-methyl-D-ribopyranose.

Low polar resulting product (3.5 mg, 15%)

$^1H$ NMR(400MHz, $CDCl_3$) δ

3.42 (3H, s, OMe)
3.53 (1H, d, J=3.0Hz, H-2)
3.57 (1H, ddd, J=2.7, 4.8, 10.8Hz, H-4)
3.63 (3H, s, COOMe)
3.70 (1H, ddd, J=0.9, 4.8, 10.8Hz, H-5)
3.98 (1H, dd, J=10.4, 10.8Hz, H-5)
4.17 (1H, dd, J=2.7, 3.0Hz, H-3)
4.32 (1H, d, J=12.3Hz, -C$\underline{H}_2$Ph)
4.51 (1H, d, J=12.3Hz, -C$\underline{H}_2$Ph)
4.52 (1H, d, J=12.1Hz, -C$\underline{H}_2$Ph)
4.56 (1H, d, J=12.1Hz, -C$\underline{H}_2$Ph)
4.85 (1H, d, J=12.4Hz, -C$\underline{H}_2$Ph)
4.92 (1H, d, J=12.4Hz, -C$\underline{H}_2$Ph)
7.19-7.49 (15H, m, Ar-H)

High polar resulting product (12.0 mg, 0.024 mmol, 51%)

$^1H$ NMR(400MHz, $CDCl_3$) δ

3.20 (3H, s, OMe)
3.62 (1H, dd, J=2.0, 12.5Hz, H-5)
3.71 (3H, s, COOMe)
3.76 (1H, td, J=2.0, 3.3Hz, H-4)
3.85 (1H, t, J=3.3Hz, H-3)

4.13 (1H, d, J=3.3Hz, H-2)
4.15 (1H, dd, J=2.0, 12.5Hz, H-5)
4.57 (2H, s, -C$\underline{H}_2$Ph)
4.68 (1H, d, J=11.6Hz, -C$\underline{H}_2$Ph)
4.69 (1H, d, J=12.6Hz, -C$\underline{H}_2$Ph)
4.85 (1H, d, J=12.6Hz, -C$\underline{H}_2$Ph)
5.00 (1H, d, J=11.6Hz, -C$\underline{H}_2$Ph)
7.18-7.41 (15H, m, Ar-H)

[Example 13]

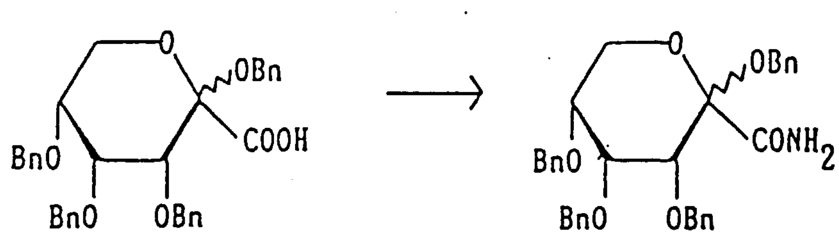

Triethylamine (0.275 ml, 2.0 mmol) was added to a solution of 1,2,3,4-tetra-O-benzyl-1-carboxy-1-dehydro-D-ribopyranose (295 mg, 2.0 mmol) in tetrahydrofuran (2 ml). The mixture was cooled to 0°C, and isopropoxycarbonyl chloride (24.4 ml, 0.21 mol) was slowly added dropwise thereto. The mixture was stirred at 0°C for 1 hour and 15 minutes and at room temperature for 45 minutes. After disappearance of the starting compounds was confirmed, ammonia gas was blown into the mixture at 0°C, followed by further stirring at room temperature for 30 minutes. The mixture was partitioned with methylene chloride and water, and the aqueous layer was extracted with methylene chloride. The organic layers were combined, washed with a saturated aqueous NaCl solution, dried over anhydrous sodium sulfate, filtered and then concentrated under reduced pressure. The residue was purified by preparative silica gel column chromatography (hexane/ethyl acetate = 6/4) to obtain 1,2,3,4-tetra-O-benzyl-1-carbamoyl-1-dehydro-D-ribopyranose as a colorless oil (233 mg, yield from benzyl 3,4,5-tri-O-benzyl-D-psicopyranoside: 85%).

$[\alpha]_D^{20}$ -5.25° (c 1.10, $CHCl_3$)
IR (neat, $cm^{-1}$)

3500, 3380, 3060, 2900, 1710, 1590, 1510, 1460, 1390, 1370, 1230, 1145, 1070

$^1$H NMR(400MHz, $CDCl_3$) δ

3.68 (1H, dd, J=12.3, 2.0Hz, H-6)
3.73-3.76 (1H, m, H-5)
3.90 (1H, t, J=3.2Hz, H-4)
4.03 (1H, dd, J=12.3, 1.8Hz, H-6)
4.29 (1H, dd, J=3.0, 1.0Hz, H-3)
4.44 (1H, d, J=11.2Hz, -C$\underline{H}_2$Ph)
4.51 (1H, d, J=11.2Hz, -C$\underline{H}_2$Ph)
4.54 (1H, d, J=12.0Hz, -C$\underline{H}_2$Ph)
4.60 (1H, d, J=12.0Hz, -C$\underline{H}_2$Ph)
4.74 (1H, d, J=11.1Hz, -C$\underline{H}_2$Ph)
4.80 (2H, s, -C$\underline{H}_2$Ph)
4.93 (1H, d, J=11.1Hz, -C$\underline{H}_2$Ph)
5.60 (1H, brd, J=2.3Hz, NH)
6.90 (1H, brd, J=2.3Hz, NH)
7.21-7.41 (20H, m)

HRMS (m/z)

| | | |
|---|---|---|
| $M^+$-Bn ($C_{27}H_{28}N_1O_6$) | Found | 462.1921 |
| | Calcd. | 462.1915 |

[Example 14]

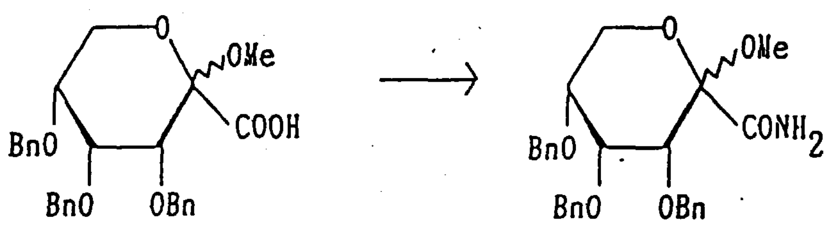

Triethylamine (20.0 μl, 0.14 mmol) was added to a solution of 2,3,4-tri-O-benzyl-1-carboxy-1-dehydro-1-O-methyl-α,β-D-ribopyranose (34.3 mg, 0.072 mmol) in tetrahydrofuran (2 ml). The mixture was cooled to 0°C. and isopropoxycarbonyl chloride (10.2 μl, 0.079 mmol) was slowly added dropwise thereto. The mixture was stirred at 0°C for 1 hour. After disappearance of the starting compounds was confirmed, ammonia gas was blown into the mixture at that temperature, and the mixture was further stirred at room temperature for 30 minutes. The mixture was partitioned with methylene chloride and water, and the aqueous layer was extracted with methylene chloride. The organic layers were combined, washed with a saturated aqueous NaCl solution, dried over anhydrous sodium sulfate, filtered and then concentrated under reduced pressure. The residue was purified by preparative silica gel thin layer chromatography (hexane/ethyl acetate = 2/8) to obtain 2,3,4-tri-O-benzyl-1-carbamoyl-1-dehydro-1-O-methyl-α,β-D-ribopyranose (both yields are total yields from methyl 3,4,5-tri-O-benzyl-α,β-D-psicopyranoside).

Low polar resulting product (22.9 mg, 67%)

$^1$H NMR(400MHz, $CDCl_3$) δ

3.22 (3H, s, OMe)
3.66 (1H, dd, J=12.3, 2.0Hz, H-5)
3.76 (1H, ddd, J=3.2, 2.0, 1.8Hz, H-4)
3.85 (1H, dd, J=3.2, 3.1Hz, H-3)
4.01 (1H, dd, J=12.3, 1.8Hz, H-5)
4.19 (1H, dd, J=3.1, 1.0Hz, H-2)
4.54 (1H, d, J=12.2Hz, -C$\underline{H}_2$Ph)
4.60 (1H, d, J=12.2Hz, -C$\underline{H}_2$Ph)
4.71 (1H, d, J=11.1Hz, -C$\underline{H}_2$Ph)
4.79 (1H, d, J=12.5Hz, -C$\underline{H}_2$Ph)
4.83 (1H, d, J=12.5Hz, -C$\underline{H}_2$Ph)
4.90 (1H, d, J=11.1Hz, -C$\underline{H}_2$Ph)
5.61 (1H, brs, NH)
6.95 (1H, brs, NH)
7.20-7.40 (15H, m, Ar-H)

High polar resulting product (4.6 mg, 13%)

$^1H$ NMR(400MHz, $CDCl_3$) δ

3.33 (3H, s, OMe)
3.44 (1H, d, J=3.3Hz, H-2)
3.58 (1H, ddd, J=10.8, 4.7, 2.6Hz, H-4)
3.77 (1H, ddd, J=10.3, 4.7, 1.0Hz, H-5)
3.99 (1H, dd, J=10.3, 10.8Hz, H-5)
4.14 (1H, dd, J=3.3, 2.6Hz, H-3)
4.42 (1H, d, J=11.6Hz, $-C\underline{H}_2Ph$)
4.53 (1H, d, J=11.6Hz, $-C\underline{H}_2Ph$)
4.53 (2H, s, $-C\underline{H}_2Ph$)
4.88 (2H, AB, $-C\underline{H}_2Ph$)
5.58 (1H, brs, NH)
6.62 (1H, brs, NH)
7.19-7.39 (15H, m, Ar-H)

[Example 15]

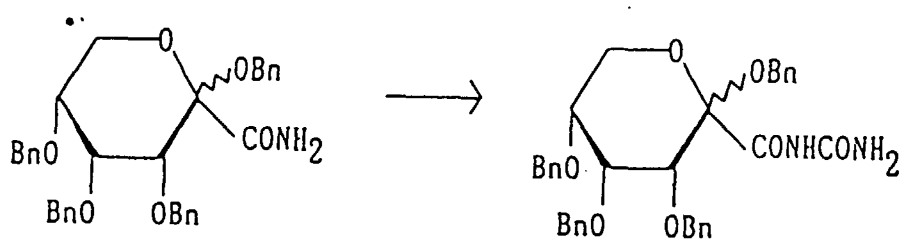

Oxalyl chloride (67.5 μl, 0.77 mmol) was added at room temperature to a solution of 1,2,3,4-tetra-O-benzyl-1-carbamoyl-1-dehydro-D-ribopyranose (121 mg, 0.22 mmol) in 1,2-dichloroethane (5 ml), and the mixture was stirred for 30 minutes. After the mixture was further stirred at 85°C for 2 hours, the reaction mixture was cooled to room temperature and stirred for 30 minutes while blowing ammonia gas thereinto. After the reaction mixture was partitioned with methylene chloride and water and the aqueous layer was extracted with methylene chloride, the organic layers were combined, washed with a saturated aqueous NaCl solution, dried over anhydrous sodium sulfate, filtered and then concentrated under reduced pressure. The residue was purified by preparative silica gel thin layer chromatography (carbon tetrachloride/ethyl ether = 3/7) to obtain 1,2,3,4-tetra-O-benzyl-1-allophanoyl-1-dehydro-D-ribopyranose as a colorless oil (16.6 g, 87%).

$[\alpha]_D^{20}$ -20.0° (c 1.99, $CHCl_3$)
IR (neat, $cm^{-1}$)

3450, 3050, 1735, 1600, 1480, 1390, 1250, 1160, 1130, 1080

$^1H$ NMR(400MHz, $CDCl_3$) δ

3.69 (1H, dd, J=12.3, 2.2Hz, H-5)
3.74-3.78 (1H, br, H-4)
3.89 (1H, t, J=3.1Hz, H-3)
4.10 (1H, dd, J=12.3, 2.4Hz, H-5)
4.17 (1H, dd, J=2.8, 1.0Hz, H-2)

4.38 (1H, d, J=11.2Hz, -C$\underline{H}_2$Ph)
4.46 (1H, d, J=11.2Hz, -C$\underline{H}_2$Ph)
4.59 (2H, s, -C$\underline{H}_2$Ph)
4.66 (1H, d, J=11.4Hz, -C$\underline{H}_2$Ph)
4.73 (1H, d, J=12.5Hz, -C$\underline{H}_2$Ph)
4.77 (1H, d, J=12.5Hz, -C$\underline{H}_2$Ph)
4.95 (1H, d, J=11.4Hz, -C$H_2$Ph)
5.19 (1H, brs, NH)
7.21-7.36 (20H, m, Ar-H)
8.00 (1H, brs, NH)
8.82 (1H, brs, NH)

HRMS (m/z)

| | | |
|---|---|---|
| $M^+$-Bn ($C_{28}H_{29}N_2O_7$) | Found | 505.1990 |
| | Calcd. | 505.1973 |

[Example 16]

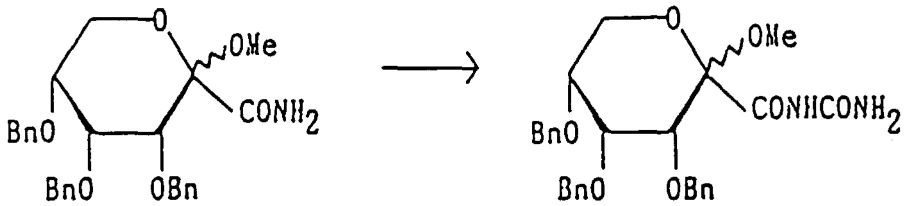

Oxalyl chloride (17.0 μl, 0.20 mmol) was added at room temperature to a solution of 2,3,4-tri-O-benzyl-1-carbamoyl-1-dehydro-1-O-methyl-α(β)-D-ribopyranose (a low polar compound) (46.5 mg, 0.098 mmol) in 1,2-dichloroethane (2 ml), and the mixture was stirred for 30 minutes. After the mixture was stirred at 90°C for 1.5 hours, the reaction mixture was cooled to room temperature. Oxalyl chloride (8.5 μl, 0.098 mmol) was then added to the mixture, followed by stirring at 90°C for 30 minutes. The reaction mixture was cooled to room temperature again and then stirred for 30 minutes while blowing ammonia gas thereinto. The reaction mixture was partitioned with methylene chloride and water. After the aqueous layer was extracted with methylene chloride, the organic layers were combined, washed with a saturated aqueous NaCl solution, dried over anhydrous sodium sulfate, filtered and then concentrated under reduced pressure. The residue was purified by preparative silica gel thin layer chromatography (hexane/ethyl acetate = 3/7) to obtain 2,3,4-tri-O-benzyl-1-allophanoyl-1-dehydro-1-O-methyl-α(β)-D-ribopyranose as a colorless oil (42.2 mg, 83%).

$^1$H NMR(200MHz, $CDCl_3$) δ

3.19 (3H, s, OMe)
3.66 (1H, dd, J=12.3, 2.1Hz, H-5)
3.71-3.79 (1H, m, H-4)
3.84 (1H, dd, J=3.2, 3.0Hz, H-3)
4.09 (1H, dd, J=12.3, 2.2Hz, H-5)
4.10 (1H, dd, J=3.0, 0.9Hz, H-2)
4.57-4.78 (5H, m, -C$\underline{H}_2$Ph)
4.94 (1H, d, J=11.4Hz, -C$\underline{H}_2$Ph)
5.17 (1H, brs, NH)
7.14-7.40 (15H, m, Ar-H)

8.00 (1H, brs, NH)
8.76 (1H, brs, NH)

[Example 17]

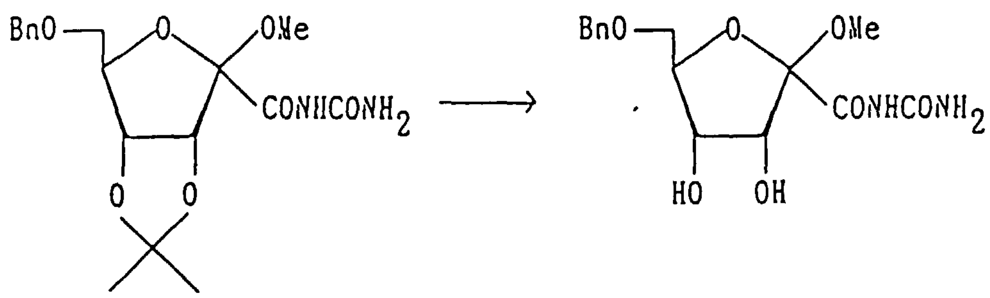

One drop of conc. hydrochloric acid was added to a solution of 5-O-benzyl-1-α-allophanoyl-1-dehydro-2,3-O-isopropylidene-1-O-methyl-β-D-ribofuranose (10.0 mg, 0.026 mmol) in methanol (2 ml). The mixture was stirred under heating at 60°C for 1.5 hours. The mixture was neutralized by adding concentrated aqueous ammonia to terminate the reaction and concentrated under reduced pressure. The residue was partitioned with ethyl acetate and water. After the aqueous layer was extracted with ethyl acetate, the organic layers were combined, washed with a saturated aqueous NaCl solution, filtered and dried over anhydrous sodium sulfate. The residue was purified by preparative silica gel thin layer chromatography (chloroform/methanol = 9/1) to obtain 5-O-benzyl-1-α-allophanoyl-1-dehydro-1-O-methyl-β-D-ribofuranose as a colorless oil (7.9 mg, 89%).

[Example 18]

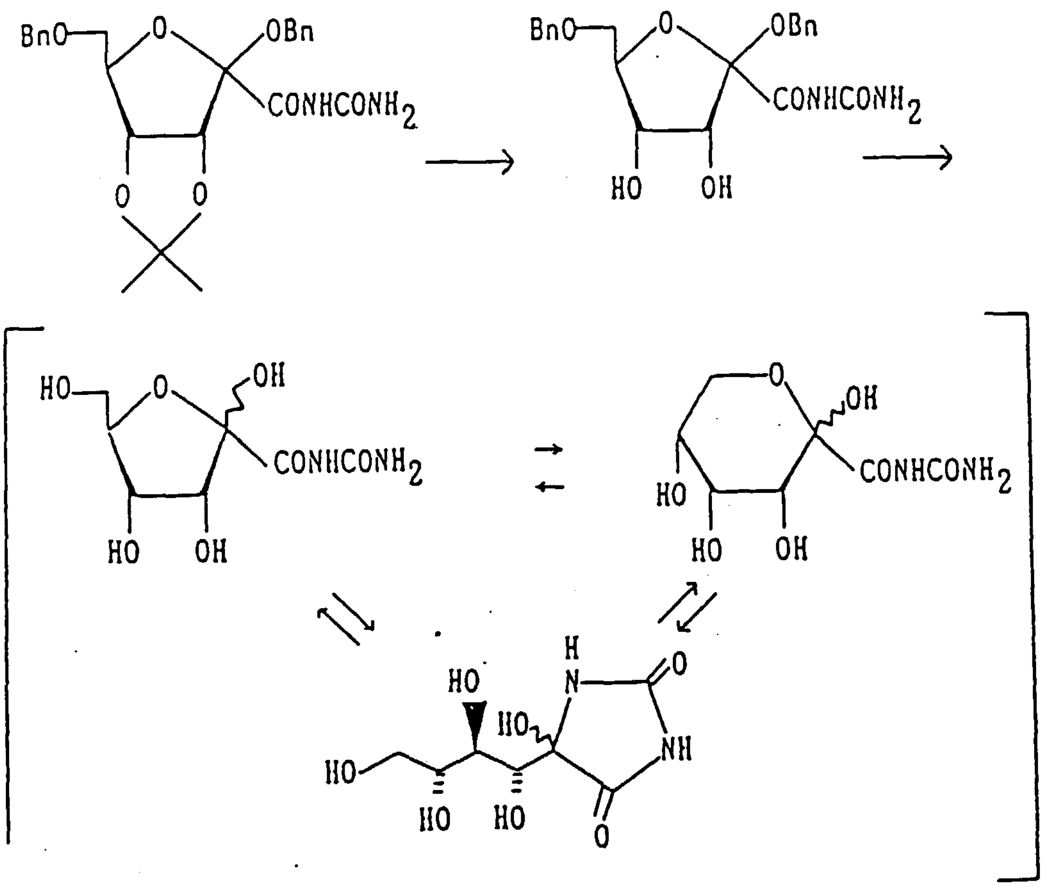

Conc. hydrochloric acid (2 ml) and water (2 ml) were added to a solution of 1,5-di-O-benzyl-1-α-allophanoyl-1-dehydro-2,3-O-isopropylidene-β-D-ribofuranose (15.8 g, 35 mmol) in ethanol (200 ml). While distilling the resulting acetone off, the mixture was stirred under heating at 90°C for 2 hours. Water (2 ml) was then added to the mixture, and the resulting mixture was stirred for 1.5 hours. Water (5 ml) was added again to the mixture, and the resulting mixture was stirred for 1.5 hours. After disappearance of the starting compounds was confirmed, the mixture was neutralized with concentrated aqueous ammonia to terminate the reaction and concentrated under reduced pressure. The residue was partitioned with ethyl acetate and water. After the aqueous layer was extracted with ethyl acetate, the organic layers were combined, washed with a saturated aqueous NaCl solution, filtered and then dried over anhydrous sodium sulfate. The residue was purified by silica gel column chromatography (hexane/ethyl acetate = 1/1 and then chloroform/methanol = 9/1) to obtain 1,5-di-O-benzyl-1-α-allophanoyl-1-dehydro-β-D-ribofuranose as a colorless oil (11.4 g, 79%).

$[\alpha]_D^{20}$ -40.7° (c 0.67. $CHCl_3$:MeOH=1:1)
IR (KBr, $cm^{-1}$)

3440, 3370, 1730, 1685, 1610, 1460, 1420, 1100

$^1$H NMR(400MHz, $CD_3OD$) δ

3.57 (1H, dd, J=10.8, 5.9Hz, H-5)
3.80 (1H, dd, J=10.8, 2.3Hz, H-5)
4.09 (1H, d, J=4.2Hz, H-2)

4.25 (1H, d, J=10.8Hz, -C$\underline{H}_2$Ph)
4.32 (1H, dd, J=8.3, 4.2Hz, H-3)
4.38 (1H, ddd, J=8.3, 5.9, 2.3Hz, H-4)
4.53-4.63 (3H, m, -C$\underline{H}_2$Ph)
7.19-7.38 (10H, m, Ar-H)

HRMS (m/z)

| | | |
|---|---|---|
| $M^+$-CONHCONH$_2$ ($C_{19}H_{21}O_5$) | Found | 329.1414 |
| | Calcd. | 329.1388 |

CIMS (m/z)

417 ($M^+$+1)

10% Palladium carbon (100 mg) was added to a solution of 1,5-di-O-benzyl-1-α-allophanoyl-1-dehydro-β-D-ribofuranose (1.00 g, 2.4 mmol) in ethanol (20 ml), and the mixture was stirred under hydrogen atmosphere at 3 atmospheric pressure for 10 hours. After filtration using Celite, the filtrate was concentrated under reduced pressure to obtain a mixture (570 mg, quantitative) of 1-allophanoyl-1-dehyro-α,β-D-ribofuranose, 1-allophanoyl-1-dehydro-α,β-D-ribopyranose.
(as a mixture of four kinds of isomers)

$^{13}$C NMR(100MHz, $D_2O$) δ

174.7 (two kinds), 174.0, 173.6 1C(-NH$\underline{C}$ONH$_2$) in total
157.8 (four kinds) (-$\underline{C}$ONHCONH$_2$)
106.8, 103.5, 99.8, 99.5 1C (anomeric position) in total

and [5RS,1'R,2'R,3'R]-5-hydroxy-5-(1',2',3',4'-tetrahydroxy)butylhydantoin

$^{13}$C NMR(100MHz, $D_2O$) δ

178.9(C-2), 161.0(C-4), 90.6(C-5), 75.7, 74.8, 72.8, 65.1(C-4')

$^{13}$C NMR(100MHz, $D_2O$) δ

178.5(C-2), 161.0(C-4), 88.9(C-5), 76.0, 75.4, 74.2, 64.5(C-4')

as a crude product [isomer ratio in the respective compounds: 6:4:3:2 in ribose derivative, 6:3 in hydantoin derivative (not in order, respectively)]. By leaving to stand for a long time, this mixture was finally converged to an equilibrium mixture of [5RS,1'R,2'R,3'R]-5-hydroxy-5-(1',2',3',4'-tetrahydroxybutyl)hydantoin (isomer ratio: 3:1, not in order).

[Example 19]

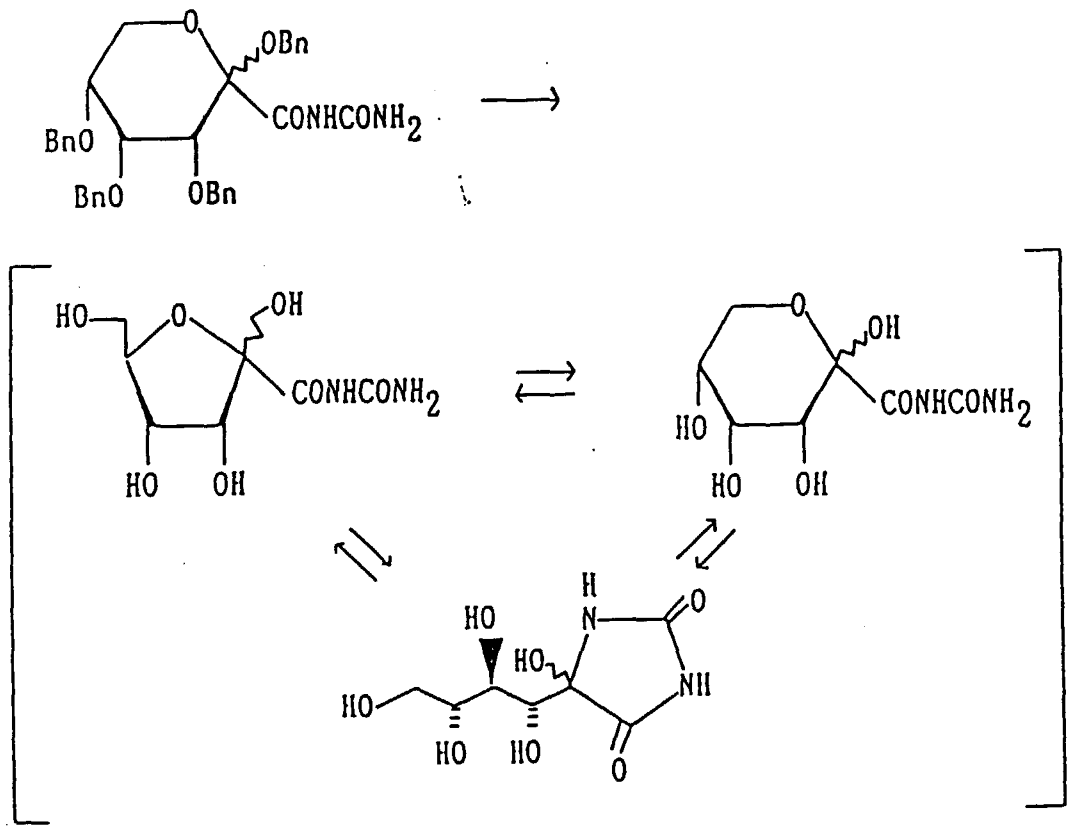

10% Palladium carbon (10 mg) was added to a solution of 1,2,3,4-tetra-O-benzyl-1-allophanoyl-1-dehydroribopyranose (10.0 mg, 0.017 mmol) in ethanol (1 ml), and the mixture was stirred under hydrogen atmosphere at 3 atmospheric pressure for 2 hours. After filtration using Celite, the filtrate was concentrated under reduced pressure to obtain an equilibrium mixture (4.3 mg, quantitative) of 1-allophanoyl-1-dehydro-α,β-D-ribofuranose, 1-allophanoyl-1-dehydro-α,β-D-ribopyranose and [5RS,1'R,2'R,3'R]-5-hydroxy-5-(1',2',3',4'-tetrahydroxybutyl)hydantoin as a crude product (isomer ratio: 6:4:3:2 in ribose derivative, 6:3 in hydantoin derivative, not in order, respectively). By leaving to stand for a long time, this compound was finally converged to an equilibrium mixture of [5RS,1'R,2'R,3'R]-5-hydroxy-5-(1',2',3',4'-tetrahydroxybutyl)hydantoin (isomer ratio: 3:1, not in order). The spectral data of the product were the same as those of Example 18.

[Reference Example 5]

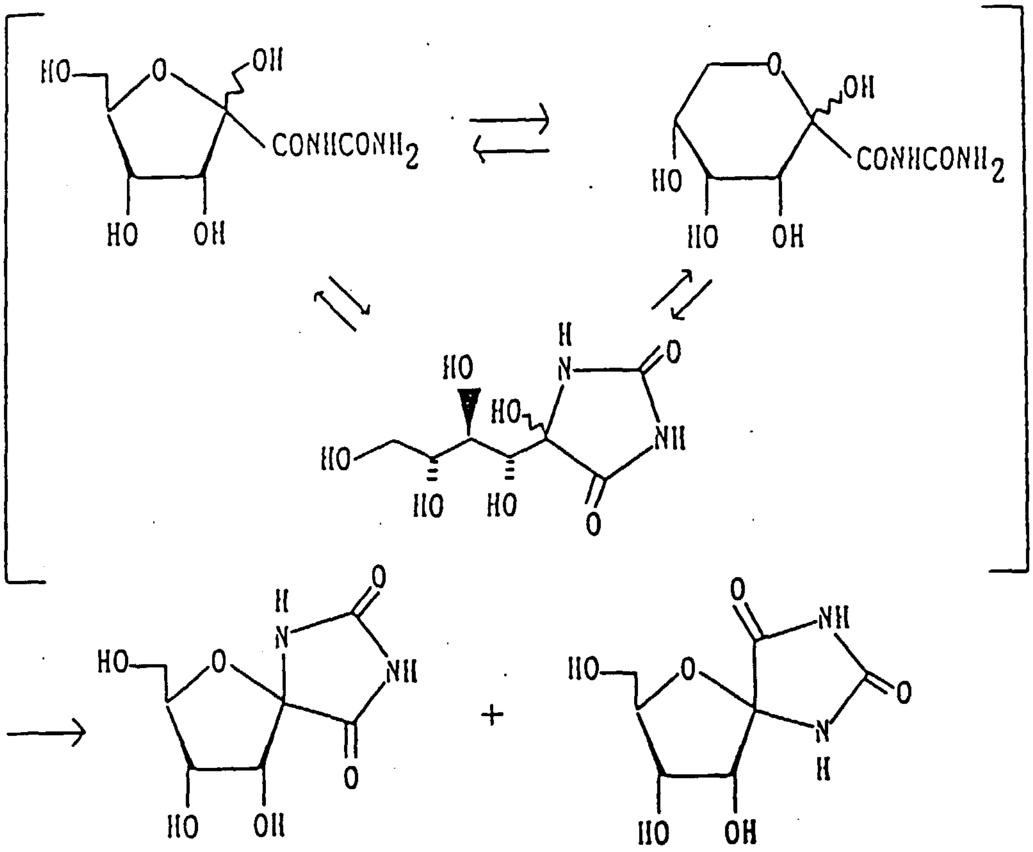

The mixture of 1-allophanoyl-1-dehydro-α,β-D-ribofuranose, 1-allophanoyl-1-dehydro-α,β-D-ribopyanose and [5RS,1'R,2'R,3'R]-5-hydroxy-5-(1',2',3',4'-tetrahydroxybutyl)hydantoin (57.3 mg, 0.24 mmol) (6:4:3:2 in ribose derivative, 6:3 in hydantoin derivtive, not in order, respectively) was dissolved in a mixed solvent of methanol and water (2:1, 6 ml). Dowex 50W (1.5 ml) was added to the solution, and the mixture was stirred vigorously at 60°C for 18 hours and further at 80°C for 2 hours. The reaction mixture was filtered to remove Dowex 50W, whereby the reaction was terminated. The filtrate was concentrated under reduced pressure to obtain a crude product (48.6 g, crude yield: 92%) containing [2R,3S,4R,5S]-3,4-dihydroxy-2-hydroxymethyl-1-oxa-6,8-diazaspiro[4.4]nonane-7,9-dione (hydantocidin) represented by the formula [I] and [2R,3S,4R,5R]-3,4-dihydroxy-2-hydroxymethyl-1-oxa-6,8-diazaspiro[4.4]nonane-7,9-dione represented by the formula [XI].

Acetic anhydride (518 mg, 0.48 mmol) and a catalytic amount of 4-dimethylaminopyridine were added to a solution of this mixture in pyridine (2 ml), followed by stirring for 30 minutes. Water was added to the reaction mixture to terminate the reaction, the mixture was partitioned with ethyl acetate and water, and then the aqueous layer was extracted with ethyl acetate. The organic layers were combined, washed successively with dilute hydrochloric acid and a saturated aqueous NaCl solution, dried over anhydrous sodium sulfate, filtered and then concentrated under reduced pressure. The resulting residue was purified by preparative silica gel thin layer chromatography (benzene/ethyl acetate = 1/1) to obtain [2R,3S,4R,5S]-3,4-diacetoxy-2-acetoxymethyl-6-N-acetyl-1-oxa-6,8-diazaspiro[4.4]nonane-7,9-dione (9.6 mg, 10.2%).

$[\alpha]_D^{20}$ +98.4° (c 1.18, $CHCl_3$)
IR (neat, $cm^{-1}$)

3250, 1815, 1760, 1380, 1310, 1240, 1110, 1050, 760

$^1H$ NMR(400MHz, $CDCl_3$) δ

2.06 (3H, s, OAc)
2.10 (3H, s, OAc)
2.14 (3H, s, OAc)
2.57 (3H, s, NAc)
4.17 (1H, dd, J=12.4, 5.9Hz, C$\underline{H}_2$OAc)
4.58 (1H, dd, J=12.4, 2.7Hz, C$\underline{H}_2$OAc)
4.62 (1H, ddd, J=8.6, 5.9, 2.7Hz, H-2)
5.46 (1H, dd, J=7.2, 8.6Hz, H-3)
5.69 (1H, d, J=7.2Hz, H-4)
7.49 (1H, brs, NH)

HRMS (m/z)

| | | |
|---|---|---|
| $M^+$ ($C_{15}H_{18}N_2O_{10}$) | Found | 387.1058 |
| | Calcd. | 387.1037 |

[2R,3S,4R,5R]-3,4-diacetoxy-2-acetoxymethyl-6-N-acetyl-1-oxa-6,8-diazaspiro[4.4]nonane-7,9-dione (18.1 mg, 19.3%),

$[\alpha]_D^{20}$ +103° (c 0.924, $CHCl_3$)
IR (neat, $cm^{-1}$)

3250, 1815, 1760, 1380, 1310, 1240, 1125, 1095, 1050, 760

$^1H$ NMR(400MHz, $CDCl_3$) δ

2.05 (3H, s, OAc)
2.14 (3H, s, OAc)
2.16 (3H, S, OAc)
2.54 (3H, s, NAc)
4.05 (1H, dd, J=12.4, 4.1Hz, C$\underline{H}_2$OAc)
4.67 (1H, dd, J=12.4, 2.7Hz, C$\underline{H}_2$OAC)
4.97 (1H, ddd, J=6.8, 4.1, 2.7Hz, H-2)
5.33 (1H, dd, J=8.6, 6.8Hz, H-3)
5.44 (1H, d, J=8.7Hz, H-4)
7.58 (1H, brs, NH)

HRMS (m/z)

| | | |
|---|---|---|
| $M^+$ ($C_{15}H_{18}N_2O_{10}$) | Found | 387.1022 |
| | Calcd. | 387.1037 |

and [2R,3S,4R,5R]-3,4-diacetoxy-2-acetoxymethyl-1-oxa-6,8-diazaspiro[4.4]nonane-7,9-dione (16.2 mg, 19.4%).

$^1H$ NMR(400MHz, $CDCl_3$) δ

2.12 (3H, s, OAc)
2.15 (3H, s, OAc)
2.16 (3H, s, OAc)
4.11 (1H, dd, J=13.5, 5.9Hz, C$\underline{H}_2$OAc)

4.41-4.47 (2H, m, H-2, C<u>H</u>$_2$OAc)
5.44 (1H, d, J=5.0Hz, H-4)
5.54 (1H, dd, J=5.0, 2.7Hz, H-3)
6.69 (1H, brs, NH-6)
8.14 (1H, brs, NH-8)

HRMS (m/z)

| | | |
|---|---|---|
| $M^{+}+1$ ($C_{13}H_{17}N_2O_9$) | Found | 345.0932 |
| | Calcd. | 345.0932 |

Methanol in which ammonia had been saturated was added to a methanol solution of these compounds, respectively. The mixtures were stirred overnight and then concentrated under reduced pressure. The residues were purified by Diaion CHP 20P to obtain [2R,3S,4R,5S]-3,4-dihydroxy-2-hydroxymethyl-1-oxa-6,8-diazaspiro[4.4]nonane-7,9-dione represented by the formula [I] (5.0 mg from (2R,3S,4R,5S]-3,4-diacetoxy-2-acetoxymethyl-6-N-acetyl-1-oxa-6,8-diazaspiro[4.4]nonane-7,9-dione, 92%) and [2R,3S,4R,5R]-3,4-dihydroxy-2-hydroxymethyl-1-oxa-6,8-diazaspiro[4.4]nonane-7,9-dione represented by the formula [XI] (7.2 mg from [2R,3S,4R,5R]-3,4-diacetoxy-2-acetoxymethyl-6-N-acetyl-1-oxa-6,8-diazaspiro[4.4]nonane-7,9-dione, 55%, 8.5 mg from [2R,3S,4R,5R]-3,4-diacetoxy-2-acetoxymethyl-1-oxa-6,8-diazaspiro[4.4]nonane-7,9-dione, 83%). The spectral data of these compounds were coincident with the values in literature (S. Mio et al., Tetrahedron, <u>47</u>, 2133, 2145 (1991)).

[I] $^{1}$H NMR(200MHz, $D_2O$) δ

3.58 (1H, dd, J=4.4, 12.7Hz, H-5)
3.69 (1H, dd, J=3.2, 12.7Hz, H-5)
4.12 (1H, dd, J=4.0, 5.8Hz, H-3)
4.20-4.30 (1H, m, H-4)
4.30 (1H, d, J=5.8Hz, H-2)

[XI] $^{1}$H NMR(400MHz, $CD_3OD$) δ

3.60 (1H, dd, J=5.2, 12.1Hz, -C<u>H</u>$_2$Oh)
3.67 (1H, dd, J=4.3, 12.1Hz, -C<u>H</u>$_2$Oh)
4.09 (1H, ddd, J=3.3, 4.3, 5.2Hz, H-2)
4.17 (1H, dd, J=3.2, 4.9Hz, H-3)
4.26 (1H, d, J=4.9Hz, H-4)

[Reference Example 6]

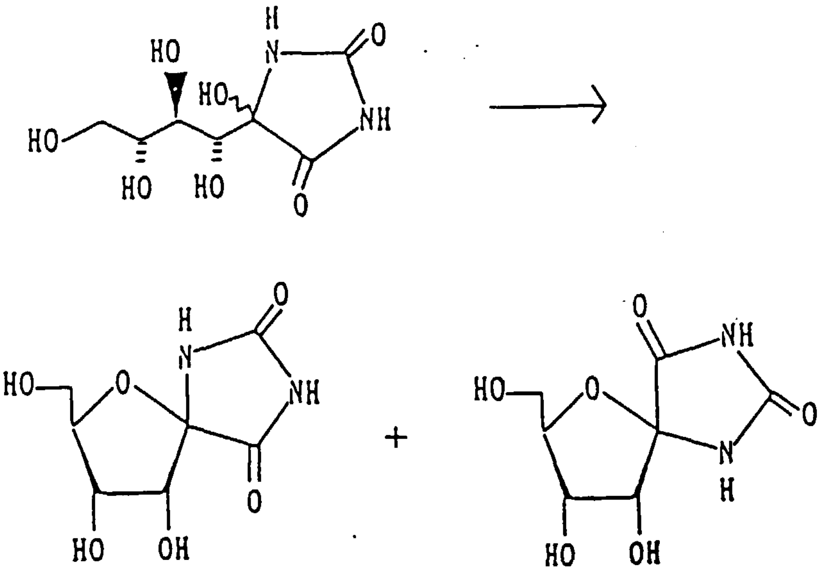

The equilibrium mixture of [5RS,1'R,2'R,3'R]-5-hydroxy-5-(1',2',3',4'-tetrahydroxybutyl)hydantoin (isomer ratio: 3:1, not in order) (101 mg, 0.43 mmol) was dissolved in a mixed solvent of methanol and water (2:1, 6 ml). Dowex 50W (3.5 ml) was added to the solution, and the mixture was stirred vigorously at 45°C for 15 hours. The reaction solution was filtered to remove Dowex 50W, whereby the reaction was terminated. The filtrate was concentrated under reduced pressure to obtain a mixture (1:4) of [2R,3S,4R,5S]-3,4-dihydroxy-2-hydroxymethyl-1-oxa-6,8-diazaspiro[4.4]nonane-7,9-dione (hydantocidin) represented by the formula [I] and [2R,3S,4R,5R]-3,4-dihydroxy-2-hydroxymethyl-1-oxa-6,8-diazaspiro[4.4]nonane-7,9-dione represented by the formula [XI] as a crude product (98.0 mg, quantitative).

Acetic anhydride (518 mg, 0.48 mmol) and a catalytic amount of 4-dimethylaminopyridine were added to a solution of this mixture in pyridine (5 ml), and the mixture was stirred for 30 minutes. Water was added to the reaction mixture to terminate the reaction, the mixture was partitioned with ethyl acetate and water, and then the aqueous layer was extracted with ethyl acetate. The organic layers were combined, washed successively with diluted hydrochloric acid and a saturated aqueous NaCl solution, dried over anhydrous sodium sulfate, filtered and then concentrated under reduced pressure. The resulting residue was purified by preparative silica gel thin layer cromatography (benzene/ethyl acetate = 1/1) to obtain [2R,3S,4R,5S]-3,4-diacetoxy-2-acetoxymethyl-6-N-acetyl-1-oxa-6,8-diazaspiro[4.4]nonane-7,9-dione (20.2 mg, 12%) and [2R,3S,4R,5R]-3,4-diacetoxy-2-acetoxymethyl-6-N-acetyl-1-oxa-6,8-diazaspiro[4.4]nonane-7,9-dione (12.5 mg, 8.5%).

The spectral data of the products were the same as those of Reference Example 5.

Methanol in which ammonia had been saturated was added to a methanol solution of these compounds, respectively. The mixtures were stirred overnight and then concentrated under reduced pressure. The residues were purified by Diaion CHP 20P to obtain [2R,3S,4R,5S]-3,4-dihydroxy-2-hydroxymethyl-1-oxa-6,8-diazaspiro[4.4]nonane-7,9-dione represented by the formula [I] (5.5 mg from [2R,3S,4R,5S]-3,4-diacetoxy-2-acetoxymethyl-6-N-acetyl-1-oxa-6,8-diazaspiro[4.4]nonane-7,9-dione, 48%) and [2R,3S,4R,5R]-3,4-dihydroxy-2-hydroxymethyl-1-oxa-6,8-diazaspiro[4.4]nonane-7,9-dione represented by the formula [XI] (6.2 mg from [2R,3S,4R,5R]-3,4-diacetoxy-2-acetoxymethyl-6-N-acetyl-1-oxa-6,8-diazaspiro[4.4]nonane-7,9-dione, 78%). The spectral data of the products were the same as those of Reference Example 5.

## Claims

**1.** A D-psicofuranose derivative represented by the following formula:

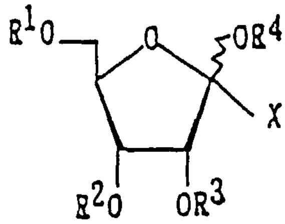

(wherein $R^1$, $R^2$, $R^3$ and $R^4$ may be the same or different from one another and each represents a hydrogen atom or a protective group for a hydroxyl group, and X represents a hydroxymethyl group, a carboxyl group which may be protected, a carbamoyl group which may be protected or an allophanoyl group which may be protected, provided that in the case where X is a hydroxymethyl group, each of $R^1$, $R^2$, $R^3$ and $R^4$ represents a protective group for a hydroxyl group).

**2.** The D-psicofuranose derivative according to Claim 1, wherein $R^2$ and $R^3$ together represent an alkylidene or arylmethylidene group represented by the following formula:

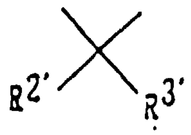

(wherein $R^{2'}$ and $R^{3'}$ may be the same or different from each other, and each represents a hydrogen atom, a straight or branched chain alkyl group having from 1 to 6 carbon atoms, a substituted or unsubstituted aryl group, or $R^{2'}$ and $R^{3'}$ may together form a ring).

**3.** The D-psicofuranose derivative according to Claim 1, wherein each of $R^1$, $R^2$, $R^3$ and $R^4$ is a hydroxyl protective group.

**4.** A D-psicopyranose derivative represented by the following formula:

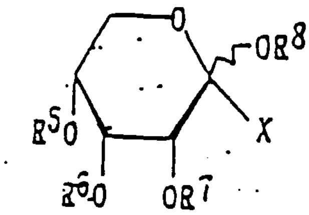

(wherein $R^5$, $R^6$, $R^7$ and $R^8$ may be the same or different from one another and each represents a hydrogen atom or a protective group for a hydroxyl group, and X represents a hydroxymethyl group, a carboxyl group which may be protected, a carbamoyl group which may be protected or an allophanoyl group which may be protected, provided that when X is a hydroxymethyl group, each of $R^5$, $R^6$, $R^7$ and $R^8$ represents a protective group for a hydroxyl group).

**5.** The D-psicopyranose derivative according to Claim 4, wherein each of $R^5$, $R^6$, $R^7$ and $R^8$ is a hydroxyl protective group.

## Patentansprüche

1. D-Psicofuranosederivat, das durch die folgende Formel dargestellt ist:

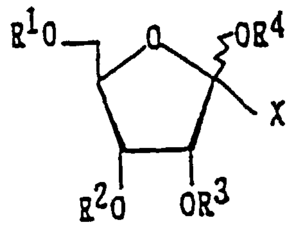

(worin $R^1$, $R^2$, $R^3$ und $R^4$ gleich oder verschieden sein können und jeweils ein Wasserstoffatom oder eine Schutzgruppe für eine Hydroxylgruppe darstellen, und X eine Hydroxymethylgruppe, eine Carboxylgruppe, die geschützt werden kann, eine Carbamoylgruppe, die geschützt werden kann oder eine Allophanoylgruppe, die geschützt werden kann, bedeutet, mit der Maßgabe, daß in dem Fall, in dem X eine Hydroxymethylgruppe ist, jedes von $R^1$, $R^2$, $R^3$ und $R^4$ eine Schutzgruppe für eine Hydroxylgruppe bedeutet).

2. D-Psicofuranosederivat nach Anspruch 1, wobei $R^2$ und $R^3$ zusammen eine Alkyliden- oder Arylmethylidengruppe darstellen, die durch die folgende Formel dargestellt ist:

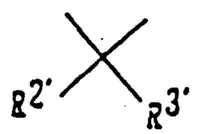

(worin $R^{2'}$ und $R^{3'}$ gleich oder verschieden sein können und jeweils ein Wasserstoffatom, eine geradekettige oder verzweigtkettige Alkylgruppe mit 1 bis 6 Kohlenstoffatomen, eine substituierte oder unsubstituierte Arylgruppe bedeuten oder $R^{2'}$ und $R^{3'}$ zusammen einen Ring bilden können).

3. D-Psicofuranosederivat nach Anspruch 1, wobei jedes von $R^1$, $R^2$, $R^3$ und $R^4$ eine Schutzgruppe für eine Hydroxylgruppe ist.

4. D-Psicofuranosederivat, das durch die folgende Formel dargestellt ist:

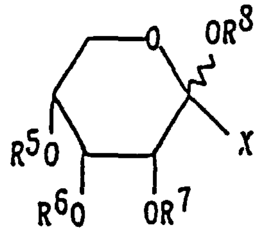

(worin $R^5$, $R^6$, $R^7$ und $R^8$ gleich oder verschieden sein können und jeweils ein Wasserstoffatom oder eine Schutzgruppe für eine Hydroxylgruppe darstellen und X eine Hydroxymethylgruppe, eine Carboxylgruppe, die geschützt werden kann, eine Carbamoylgruppe, die geschützt werden kann oder eine Allophanoylgruppe, die geschützt werden kann, darstellt, mit der Maßgabe, daß dann, wenn X eine Hydroxymethylgruppe ist, jedes von $R^5$, $R^6$, $R^7$ und $R^8$ eine Schutzgruppe für eine Hydroxylgruppe darstellt).

5. D-Psicofuranosederivat nach Anspruch 4, wobei jedes von $R^5$, $R^6$, $R^7$ und $R^8$ eine Schutzgruppe für eine Hydroxylgruppe ist.

**Revendications**

1. Dérivé D-psicofuranose représenté par la formule suivante:

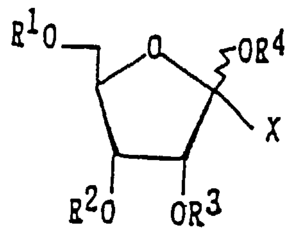

(dans laquelle $R^1$, $R^2$, $R^3$ et $R^4$ peuvent être identiques ou différents les uns des autres, et chacun représente un atome d'hydrogène ou un groupe protecteur pour un groupe hydroxyle, et X représente un groupe hydroxyméthyle, un groupe carboxyle qui peut être protégé, un groupe carbamoyle qui peut être protégé ou un groupe allophanoyle qui peut être protégé, à condition que, lorsque X est un groupe hydroxymethyle, chacun des radicaux $R^1$, $R^2$, $R^3$ et $R^4$ représente un groupe protecteur pour un groupe hydroxyle).

2. Dérivé D-psicofuranose selon la revendication 1, dans lequel $R^2$ et $R^3$ représentent ensemble un groupe alkylidène ou un groupe arylméthylidène représenté par la formule suivante:

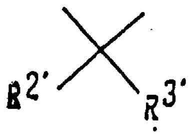

(dans laquelle $R^{2'}$ et $R^{3'}$ peuvent être identiques ou différents les uns des autres, et chacun représente un atome d'hydrogène, un groupe alkyle à chaîne droite ou ramifiée comportant 1 à 6 atomes de carbone, un groupe aryle substitué ou non substitué, ou bien $R^{2'}$ et $R^{3'}$ peuvent ensemble former un cycle).

3. Dérivé D-psicofuranose selon la revendication 1, dans lequel chacun des radicaux $R^1$, $R^2$, $R^3$ et $R^4$ est un groupe protecteur de fonction hydroxyle.

4. Dérivé D-psicopyranose représenté par la formule suivante:

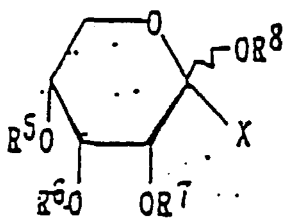

(dans laquelle $R^5$, $R^6$, $R^7$ et $R^8$ peuvent être identiques ou différents les uns des autres, et chacun représente un atome d'hydrogène ou un groupe protecteur pour un groupe hydroxyle, et X représente un groupe hydroxyméthyle, un groupe carboxyle qui peut être protégé, un groupe carbamoyle qui peut être protégé ou un groupe allophanoyle qui peut être protégé, à condition que, lorsque X est un groupe hydroxyméthyle, chacun des radicaux $R^5$, $R^6$, $R^7$ et $R^8$ représente un groupe protecteur pour un groupe hydroxyle).

5. Dérivé D-psicopyranose selon la revendication 4, dans lequel chacun des radicaux $R^5$, $R^6$, $R^7$ et $R^8$ est un groupe protecteur de fonction hydroxyle.